(19) Europäisches Patentamt
European Patent Office
Office européen des brevets

(11) EP 3 376 869 B1

# (12) EUROPEAN PATENT SPECIFICATION

(45) Date of publication and mention of the grant of the patent:
**05.01.2022 Bulletin 2022/01**

(21) Application number: **16866909.1**

(22) Date of filing: **14.11.2016**

(51) Int Cl.:
***A01N 43/90*** *(2006.01)*
***C07D 491/00*** *(2006.01)*
***C07D 487/04*** *(2006.01)*
***A61K 31/506*** *(2006.01)*
***A61P 19/04*** *(2006.01)*
***A61P 25/00*** *(2006.01)*
***A61P 37/06*** *(2006.01)*
***A61P 17/06*** *(2006.01)*
***A61P 19/02*** *(2006.01)*

(86) International application number:
**PCT/US2016/061824**

(87) International publication number:
**WO 2017/087318 (26.05.2017 Gazette 2017/21)**

## (54) TREATMENT OF AUTOIMMUNE DISEASE

BEHANDLUNG DER AUTOIMMUNERKRANKUNG

TRAITEMENT DE MALADIE AUTO-IMMUNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.11.2015 US 201562258190 P**

(43) Date of publication of application:
**26.09.2018 Bulletin 2018/39**

(73) Proprietor: **Mingsight Pharmaceuticals, Inc.**
**San Diego, CA 92196 (US)**

(72) Inventors:
- **NIESMAN, Michael**
  **San Diego, CA 92196 (US)**
- **ZHANG, Kai**
  **San Diego, CA 92196 (US)**

(74) Representative: **Mewburn Ellis LLP**
**Aurora Building**
**Counterslip**
**Bristol BS1 6BX (GB)**

(56) References cited:
**WO-A1-2015/019325**
**WO-A1-2015/157955**
**US-A1- 2005 101 594**
**US-A1- 2007 004 705**
**US-A1- 2007 004 705**
**US-A1- 2010 130 501**
**US-A1- 2010 210 622**
**US-A1- 2015 072 986**
**US-A1- 2015 099 743**
**US-A1- 2015 099 743**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 376 869 B1

## Description

### BACKGROUND

A need exists in the medical art for compounds and methods for the treatment of rheumatoid arthritis and other autoimmune and alloimmune mediated diseases and conditions.

US 2015/0099743 A1 (09 April 2015) describes certain *N*-pyrimidin-4-yl-3-amino-pyrrolo[3,4-C]pyrazole derivatives as PKC Kinase inhibitors and their use in methods of treating diabetes mellitus and its complications, cancer, ischemia, inflammation, central nervous system disorders, cardiovascular disease, Alzheimer's disease and dermatological disease pression, virus diseases, inflammatory disorders, and diseases in which the liver is a target organ.

US 2007/0004705 A1 (04 January 2007) describes certain pyrrolo-pyrazole derivatives as kinase inhibitors and their use in the treatment of cell cycle proliferatuve disorders associated with an altered cell cycle dependent kinase activity.

### SUMMARY OF THE INVENTION

The invention is a pharmaceutical composition comprising 5-{[(2S,5R)-2,5-Dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof,
for use in a method of treating an autoimmune or alloimmune disease in a patient, wherein the autoimmune or alloimmune disease is:

rheumatoid arthritis;
multiple sclerosis;
inflammatory bowel disease;
optic neuritis;
neuromyelitis optica;
Sjögren's syndrome;
systemic scleroderma;
ankylosing spondylitis;
autoimmune hepatitis;
graft vs host disease; or
organ transplant rejection.

In one embodiment, the autoimmune or alloimmune disease is rheumatoid arthritis. In one embodiment, the rheumatoid arthritis is rheumatoid factor positive (seropositive) RA, rheumatoid factor negative (seronegative) RA, or juvenile RA

In one embodiment, the autoimmune or alloimmune disease is multiple sclerosis. In one embodiment, the multiple sclerosis is relapsing-remitting (RR) multiple sclerosis, secondary progressive (SP) multiple sclerosis, primary progressive (PP) multiple sclerosis, progressive relapsing multiple sclerosis, clinically isolated syndrome (CIS), or radiologically isolated syndrome (RIS).

In one embodiment, the autoimmune or alloimmune disease is inflammatory bowel disease. In one embodiment, the inflammatory bowel disease is Crohn's disease, ulcerative colitis, collagenous colitis, lymphocytic colitis, diversion colitis, Behçet's disease, or indeterminate colitis.

In one embodiment, the autoimmune or alloimmune disease is optic neuritis; wherein the optic neuritis is papillitis neuritis or retrobulbar neuritis.

In one embodiment, the autoimmune or alloimmune disease is neuromyelitis optica.

In one embodiment, the autoimmune or alloimmune disease is Sjögren's syndrome.

In one embodiment, the autoimmune or alloimmune disease is systemic scleroderma.

In one embodiment, the autoimmune or alloimmune disease is ankylosing spondylitis.

In one embodiment, the autoimmune or alloimmune disease is autoimmune hepatitis.

In one embodiment, the autoimmune or alloimmune disease is graft vs host disease; wherein the graft vs host disease is acute graft vs host disease, or chronic graft vs host disease.

In one embodiment, wherein the autoimmune or alloimmune disease is organ transplant rejection.

### BRIEF DESCRIPTION

Described herein are compositions and methods for the treatment of autoimmune and alloimmune diseases

and conditions, including rheumatoid arthritis, multiple sclerosis, inflammatory bowel disease (IBD) (e.g., Crohn's disease, ulcerative colitis), optic neuritis, neuromyelitis optica, Sjögren's syndrome, systemic scleroderma, ankylosing spondylitis, autoimmune hepatitis, Graft vs host disease (GvHD), and organ transplant rejection. Said compositions useful for treating autoimmune diseases comprise pyrrolo-pyrazole PKC inhibitors.

Also described herein is a method of treating rheumatoid arthritis in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

Also described herein is a method of treating multiple sclerosis in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

Also described herein is a method of treating inflammatory bowel disease (IBD) in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

Also described herein is a method of treating Crohn's disease in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

Also described herein is a method of treating ulcerative colitis in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

Also described herein is a method of treating optic neuritis in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

Also described herein is a method of treating neuromyelitis optica in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

Also described herein is a method of treating Sjögren's syndrome in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

Also described herein is a method of treating systemic scleroderma in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

Also described herein is a method of treating ankylosing spondylitis in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

Also described herein is a method of treating autoimmune hepatitis in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

Also described herein is a method of treating Graft vs host disease (GVHD) in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

Also described herein is a method of treating organ transplant rejection in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

Also described herein is a method of treating autoimmune and alloimmune diseases and conditions, including rheumatoid arthritis, multiple sclerosis, inflammatory bowel disease (IBD) (e.g., Crohn's disease, ulcerative colitis), optic neuritis, neuromyelitis optica, Sjögren's syndrome, systemic scleroderma, ankylosing spondylitis, autoimmune hepatitis, Graft vs host disease (GvHD), and organ transplant rejection, in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

## BRIEF DESCRIPTION OF THE DRAWINGS

Figure 1 illustrates the body weight change in grams from day 17 to day 35 in a rheumatoid arthritis mouse model;
Figure 2 illustrates the percent change in body weight from baseline day 17 in a rheumatoid arthritis mouse model;
Figure 3 illustrates the body weight change in grams from study day 17 in a rheumatoid arthritis mouse model;
Figure 4 illustrates the clinical arthritis score for all paws in a rheumatoid arthritis mouse model;
Figure 5 illustrates the clinical arthritis score with AUC calculation for all paws in a rheumatoid arthritis mouse model; and
Figure 6 illustrates the percent incidence over time in a rheumatoid arthritis mouse model.
Figure 7 illustrates efficacy of compound A in reducing urine score in a MRL/lpr lupus model;
Figure 8 illustrates efficacy of compound A in reducing lymphadenopathy in a MRL/lpr lupus model;
Figure 9 illustrates efficacy of compound A in reducing splenomegaly in a MRL/lpr lupus model;
Figure 10 illustrates efficacy of compound A in treatment of increased spleen weight;
Figure 11 illustrates efficacy of compound A in a rat model of encephalitis;
Figure 12 illustrates clinical scores after administration of compound A in a rat uveitis model; and
Figure 13 illustrates histological scores of compound A in a rat uveitis model.

## DETAILED DESCRIPTION

### Certain Terminology

Unless defined otherwise, all technical and scientific terms used herein have the same meaning as is commonly understood by one of skill in the art to which the claimed subject matter belongs. It is to be understood that the foregoing general description and the following detailed description are exemplary and explanatory only and are not restrictive of any subject matter claimed. In this application, the use of the singular includes the plural unless specifically stated otherwise. It must be noted that, as used in the specification and the appended claims, the singular forms "a," "an" and "the" include plural referents unless the context clearly dictates otherwise. In this application, the use of "or" means "and/or" unless stated otherwise. Furthermore, use of the term "including" as well as other forms, such as "include", "includes," and "included," is not limiting.

As used herein, ranges and amounts can be expressed as "about" a particular value or range. About also includes the exact amount. Hence "about 5 μg" means "about 5 μg" and also "5 μg." Generally, the term "about" includes an amount that would be expected to be within experimental error.

As used herein, the terms "comprising" and "including" are used in their open, non-limiting sense.

A "solvate" is intended to mean a pharmaceutically acceptable solvate form of a specified compound that retains the biological effectiveness of such compound. Examples of solvates include compounds in combination with water, isopropanol, ethanol, methanol, DMSO (dimethylsulfoxide), ethyl acetate, acetic acid, or ethanolamine.

The phrase "pharmaceutically acceptable salt(s)", as used herein, unless otherwise indicated, includes salts of acidic or basic groups which may be present in a specified compound. A compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine is basic in nature and is capable of forming a wide variety of salts with various inorganic and organic acids. The acids that may be used to prepare pharmaceutically acceptable acid

addition salts of such basic compounds are those that form non-toxic acid addition salts, i.e., salts containing pharmacologically acceptable anions, such as the acetate, benzenesulfonate, benzoate, bicarbonate, bisulfate, bitartrate, borate, bromide, calcium edetate, camsylate, carbonate, chloride, clavulanate, citrate, dihydrochloride, edetate, edislyate, estolate, esylate, ethylsuccinate, fumarate, gluceptate, gluconate, glutamate, glycollylarsanilate, hexylresorcinate, hydrabamine, hydrobromide, hydrochloride, iodide, isothionate, lactate, lactobionate, laurate, malate, maleate, mandelate, mesylate, methylsulfate, mucate, napsylate, nitrate, oleate, oxalate, pamoate (embonate), palmitate, pantothenate, phospate/diphosphate, polygalacturonate, salicylate, stearate, subacetate, succinate, tannate, tartrate, teoclate, tosylate, triethiodode, and valerate salts.

The term "treating", as used herein, unless otherwise indicated, means reversing, alleviating, inhibiting the progress of, or preventing the disorder or condition to which such term applies, or one or more symptoms of such disorder or condition. The term "treatment", as used herein, unless otherwise indicated, refers to the act of treating as "treating" is defined immediately above.

The phrase "therapeutically effective amount", as used herein, refers to that amount of drug or pharmaceutical agent that will elicit the biological or medical response of a tissue, system, animal, or human that is being sought by a researcher, veterinarian, medical doctor or other.

A compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine may also exist as tautomers. A reference to the compound includes all such tautomers and mixtures thereof.

A reference to a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine includes corresponding isotopically-labelled compounds, which are identical to that compound, but for the fact that one or more atoms are replaced by an atom having an atomic mass or mass number different from the atomic mass or mass number usually found in nature. Examples of isotopes that can be incorporated into the compounds include isotopes of hydrogen, carbon, nitrogen, oxygen, phosphorous, fluorine and chlorine, such as $^{2}H$, $^{3}H$, $^{13}C$, $^{14}C$, $^{15}N$, $^{18}O$, $^{17}O$, $^{31}P$, $^{32}P$, $^{35}S$, $^{18}F$, and $^{36}Cl$, respectively. Certain isotopically-labelled compounds, for example those into which radioactive isotopes such as $^{3}H$ and $^{14}C$ are incorporated, are useful in drug and/or substrate tissue distribution assays. Tritiated, i.e., $^{3}H$, and carbon-14, i.e., $^{14}C$, isotopes are particularly preferred for their ease of preparation and detectability. Further, substitution with heavier isotopes such as deuterium, i.e., $^{2}H$, can afford certain therapeutic advantages resulting from greater metabolic stability, for example increased *in vivo* half-life or reduced dosage requirements and, hence, may be preferred in some circumstances. Isotopically labeled compounds can generally be prepared by carrying out the procedures disclosed in the Schemes and/or in the Examples below, by substituting a readily available isotopically labeled reagent for a non-isotopically labeled reagent.

The term "mmol", as used herein, unless otherwise indicated, is intended to mean millimole. The term "equiv", as used herein, unless otherwise indicated, is intended to mean equivalent. The term "mL", as used herein, unless otherwise indicated, is intended to mean milliliter. The term "U", as used herein, unless otherwise indicated, is intended to mean units. The term "mm" as used herein, unless otherwise indicated, is intended to mean millimeter. The term "g", as used herein, unless otherwise indicated, is intended to mean gram. The term "kg", as used herein, unless otherwise indicated, is intended to mean kilogram. The term "h", as used herein, unless otherwise indicated, is intended to mean hour. The term "min", as used herein, unless otherwise indicated, is intended to mean minute. The term "μL", as used herein, unless otherwise indicated, is intended to mean microliter. The term "μM", as used herein, unless otherwise indicated, is intended to mean micromolar. The term "μm", as used herein, unless otherwise indicated, is intended to mean micrometer. The term "M", as used herein, unless otherwise indicated, is intended to mean molar. The term "N", as used herein, unless otherwise indicated, is intended to mean normal. The term "nm", as used herein, unless otherwise indicated, is intended to mean nanometer. The term "nM", as used herein, unless otherwise indicated, is intended to mean nanoMolar. The term "amu", as used herein, unless otherwise indicated, is intended to mean atomic mass unit. The term "°C", as used herein, unless otherwise indicated, is intended to mean Celsius. The term "m/z", as used herein, unless otherwise indicated, is intended to mean, mass/charge ratio. The term "wt/wt", as used herein, unless otherwise indicated, is intended to mean weight/weight. The term "v/v", as used herein, unless otherwise indicated, is intended to mean volume/volume. The term "mL/min", as used herein, unless otherwise indicated, is intended to mean milliliter/minute. The term "UV", as used herein, unless otherwise indicated, is intended to mean ultraviolet. The term "APCI-MS", as used herein, unless otherwise indicated, is intended to mean atmospheric pressure chemical ionization mass spectroscopy. The term "HPLC", as used herein, unless otherwise indicated, is intended to mean high performance liquid chromatograph. The chromatography was performed at a temperature of about 20 °C, unless otherwise indicated. The term "LC", as used herein, unless otherwise indicated, is intended to mean liquid chromatograph. The term "LCMS", as used herein, unless otherwise indicated, is intended to mean liquid chromatography mass spectroscopy. The term "TLC", as used herein, unless otherwise indicated, is intended to mean thin layer chromatography. The term "SFC", as used herein, unless otherwise indicated, is intended to mean supercritical fluid chromatography. The term "sat" as used herein, unless otherwise indicated, is intended to mean saturated. The term "aq" as used herein, is intended to mean

aqueous. The term "ELSD" as used herein, unless otherwise indicated, is intended to mean evaporative light scattering detection. The term "MS", as used herein, unless otherwise indicated, is intended to mean mass spectroscopy. The term "HRMS (ESI)", as used herein, unless otherwise indicated, is intended to mean high-resolution mass spectrometry (electrospray ionization). The term "Anal.", as used herein, unless otherwise indicated, is intended to mean analytical. The term "Calcd", as used herein, unless otherwise indicated, is intended to mean calculated. The term "N/A", as used herein, unless otherwise indicated, is intended to mean not tested. The term "RT", as used herein, unless otherwise indicated, is intended to mean room temperature. The term "Mth.", as used herein, unless otherwise indicated, is intended to mean Method. The term "Celite®", as used herein, unless otherwise indicated, is intended to mean a white solid diatomite filter agent commercially available from World Minerals located in Los Angeles, California USA. The term "Eg.", as used herein, unless otherwise indicated, is intended to mean example.

The term "Ki", as used herein, unless otherwise indicated, is intended to mean values of enzyme inhibition constant. The term "$K_i$ app", as used herein, unless otherwise indicated, is intended to mean $K_i$ apparent. The term "$IC_{50}$", as used herein, unless otherwise indicated, is intended to mean concentrations required for at least 50% enzyme inhibition.

Other aspects, advantages, and features of the invention will become apparent from the detailed description below.

**Protein Kinase C**

The superfamily of kinases known as protein kinase C (PKC) are important kinases that are active in and that act as regulators in many cell signaling pathways. (Newton, 2001, Chem. Rev. 101, 2353-2364). Specific isoforms of PKC have been implicated in the response to hyperglycemia (e.g., PKCβ (beta) Das Evcimen and King, 2007, Pharmacol Res,. 55(6): p. 498-510) and in T and B cell survival and function (e.g., PKCθ (theta): Sun, Z. 2012, Front Immunol 3, 225; PKCβ: Leitges, M. et al., 1996, Science 273, 788-791; PKCα (alpha): Gruber, T. et al., 2009, Mol Immunol 46, 2071-2079).

Both T lymphocytes and B lymphocytes (T cells and B cells) have been shown to contribute to autoimmune disease, often simultaneously (Wahren-Herlenius and Dörner T. 2013, Lancet. 382:819-31). Recent scientific reports have revealed that specific isoforms of PKC are crucial to the normal function of T and B cells and in their contribution to autoimmune disease.

Three isoforms, PKCθ, PKCα and PKCβ, appear to be most important for lymphocyte function. PKCθ is critical to T-cell function (Sun, 2012, Front Immunol 3, 225). Specifically, PKCθ is downstream of the T cell receptor complex and plays a critical role in T cell survival, function and autoimmune stimulation. Mouse models of autoimmune diseases have been used to illustrate PKCθ function in T cell-dependent autoimmunity (Marsland, B.J. and Kopf, M., 2008, Trends Immunol, 29(4) 179-85). PKCα plays a non-redundant role in T cell activation (Gruber, T., et al, 2009, Mol Immunol 46, 2071-2079; Pfeifhofer, C., et al, 2006, J Immunol 176, 6004-6011; von Essen, M., et al, 2006, J Immunol 176, 7502-75). And PKCβ plays a key role in B cell survival, function, and the dysfunction seen in autoimmunity (Leitges, M., et al, 1996, Science 273, 788-791; Saijo, K., et al, 2002, J Exp Med 195, 1647-1652; Su, T.T., et al., 2002, Nat Immunol 3, 780-786). Finally, it has been shown in mice that inhibition of PKCδ (delta) appears to have the potential to induce autoimmune disease in B cells. PKCδ knockout mice *($PKC\delta^{-/-}$)* have increased antibody production including auto-antibodies and actually display autoimmune phenotypes. (Mecklenbrauker, I., et al, 2002, Nature 416, 860-865; Miyamoto, A., et al., 2002, Nature 416, 865-869).

**Pyrrolo-pyrazole PKC Inhibitors**

The pyrrolo-pyrazole PKC inhibitors used herein have been previously described in WO 2008/096260 and WO 2008/125945 and related patents and patent applications, e.g. US 8,183,255, US 8,877,761, US patent application 14/506,470, US 8,114,871, and US 8,999,981. As used herein, the term compound A (or cmpd A) refers to 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, which was disclosed in WO 2008/096260 and has the chemical structure:

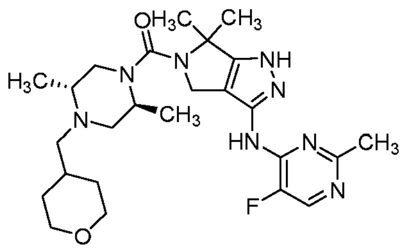

.

## Autoimmune Disease

*Rheumatoid Arthritis*

Rheumatoid arthritis (RA) is a chronic autoimmune disorder in which the body's immune system attacks the joints and additional organs such as skin, eyes, lungs, and blood vessels. In some instances, symptoms of RA include pain, swollen and/or stiffness of the joints, rheumatoid nodules, low red blood cells, and inflammation around the lungs and heart.

In some instances, RA is further classified into rheumatoid factor positive (seropositive) RA, rheumatoid factor negative (seronegative) RA, and juvenile RA (or juvenile idiopathic arthritis). Rheumatoid factor (RF) is an autoantibody directed against the Fc region of IgG. In some cases, rheumatoid factor comprises one or more isotype of immunoglobulin, such as for example, IgA, IgG, IgM, IgE, or IgD. In some cases, rheumatoid factor also includes a cryoglobulin, an antibody that precipitates at temperatures below normal body temperature. Presence or absence of rheumatoid factor (i.e., seropositive or seronegative) is used as part of a diagnostic tool in evaluating the presence and progression of RA. Juvenile RA affects children under age 16 in which the inflammation duration last more than 6 weeks.

In some embodiments, both Th17 and Th1 have been implicated in the development and progression of RA. For example, overexpression of IL-17 by Th17 cells leads to synovial inflammation, cartilage destruction, and bone erosion. Furthermore, IL-17 triggers human synoviocytes to produce IL-6, IL-8 GM-CSF, and PGE2, and triggers the production of TNF-$\alpha$, IL-1$\beta$, IL-12, stromelysin, IL-10, and IL-1R antagonist in human peripheral blood macrophages. In some instances, Th17 cells have also been observed to coexpress the Th1 cytokine IFN-$\gamma$ in peripheral blood, suggesting a plasticity of Th17 cells given rise to Th1 cells. (Nistala, et al., "Th17 plasticity in human autoimmune arthritis is driven by the inflammatory environment," PNAS 107(33):14751-14756 (2010))

In some cases, PKC (e.g., PKC-$\theta$) has been implicated in mounting a Th-1 dependent response. Indeed, PKC-$\theta$-deficient mice exhibit decreased disease severity in both mBSA-induced arthritis and collagen-induced arthritis (CIA) mouse models, reduced proliferative capacity of PKC-$\theta$-deficient T cells in response to Ag and decreased IL-2 levels, impaired expression of T-bet, and reduced levels of IFN-$\gamma$ and IL-4. Furthermore, PKC-$\theta$ deficiency correlates to a reduced T cell proliferation, Th1/Th2 cell differentiation, and T cell activation before and during disease peak. (Healy, et al., "PKC-$\theta$-deficient mice are protected from Th1-dependent antigen-induced arthritis," J Immunol 177:1886-1893 (2006))

In some embodiments, treatment of RA include disease-modifying antirheumatic drugs (DMARDs) such as methotrexate, hydroxychloroquine, sulfasalazine, leflunomide, abatacept, or anakinra; biologics such as tumor necrosis factor alpha blockers (e.g., infliximab), interleukin 1 blockers (e.g., anakinra), monoclonal antibodies (e.g., rituximab, tocilizumab), T cell costimulation blockers (e.g., abatacept); nonsteroidal anti-inflammatory drugs (NSAIDs); COX-2 inhibitors (e.., celecoxib); glucocorticoids; or surgery.

*Multiple Sclerosis*

Multiple Sclerosis (MS), also known as disseminated sclerosis or encephalomyelitis disseminata, is a demyelinating disease in which the myelin sheath of neurons, or the fatty sheath that surrounds and insulates nerve fibers in the brain and spinal cord, is damaged. In some instances, symptoms of MS include numbness or weakness of one or more portions of the body, partial or complete loss of vision, prolonged double vision, tingling or pain, Lhermitte sign, tremor, slurred speech, fatigue, dizziness, and impaired bowel and bladder functions.

In some embodiments, there are several phenotypes or disease course associated with MS. In some instances, these include relapsing-remitting (RR), secondary progressive (SPMS), primary progressive (PPMS), progressive relapsing, clinically isolated syndrome (CIS), and radiologically isolated syndrome (RIS). In some cases, the relapsing-remitting subtype begins with a clinically isolated syndrome (CIS). CIS is an attack suggestive of demyelination but does not fulfill the criteria for MS. Secondary progressive (SP) MS is characterized by a progressive neurologic decline between acute attacks without a definite period of remission. In some instances, about 65% of those with relapsing-remitting MS

progresses into SPMS. Primary progressive (PP) MS is characterized by progression of disability from onset, with no or occasional and minor remissions and improvements. Progressive relapsing MS is characterized by a steady neurologic decline with clear superimposed attacks.

In some embodiments, both B cells and T cells play a role in the development and progression of MS. For example, deregulation of pro-inflammatory cytokines such as Th1 cytokine IFNγ leads to a disruption of the blood brain barrier (BBB) (Compston, A. and Coles, A. "Multiple sclerosis," Lancet 372:1502-1517 (2008)). Furthermore, secretion of IL-17 and IL-22 by Th17 cells increases permeability of the BBB by disruption of the endothelial tight junction and by interaction with endothelium to allow further recruitment of CD4+ subsets (Hoglund, R.A., and Maghazachi, A.A. "Multiple sclerosis and the role of immune cells," World J. Exp Med. 4(3):27-37 (2014)). As such, the presence of pro-inflammatory cytokines leads to complement deposition and opsonization of the surrounding tissues of the perivascular space and parenchyma, local activation of microglia and macrophages causing demyelination, and neuronal cell death (Prineas, J.W., and Graham, J.S. "Multiple sclerosis: capping of surface immunoglobulin G on macrophages engaged in myelin breakdown." Ann Neurol. 10:149-158 (1981)). In some instances, B cells further contribute to the pathology of MS through antigen presentation, cell interactions and/or production of immunoglobulins from plasma cells (Hestvik, A.L. "The double-edged sword of autoimmunity: lessons from multiple sclerosis," Toxins 2:856-877 (2010)).

In some instances, T cell activation requires T cell receptor (TCR) interaction with MHC-peptide complexes in parallel with engagement of costimulatory molecules such as CD28. In some cases, PKC-θ is associated with TCR- and CD28-specific signals leading to T cell activation, proliferation, and cytokine production. Indeed, a study has shown that PKC-θ is important for the development of Ag-specific Th1 cells in experimental allergic encephalomyelitis (EAE), a mouse model of MS (Salek-Ardakani, et al., "Protein kinase Cθ controls Th1 cells in experimental autoimmune encephalomyelitis," J Immunol 175:7635-7641 (2005)).

PKC theta is involved in modulating both Th1 and Th2 type responses. For example, in a MOG-induced EAE model of MS, a Th1-based model, mice deficient in PKC theta were protected from disease development. Furthermore, Th-1 cytokines such as IL-2 and IFNγ were observed to decrease in the absence of PKC theta. (Anderson, et al., "Mice deficient in PKC theta demonstrate impaired in vivo T cell activation and protection from T cell-mediated inflammatory diseases," Autoimmunity, 39(6): 469-478 (2006))

PKC-θ is involved in the regulation of multiple T cell functions that are necessary for the development of autoimmune diseases. PKC-θ ablation leads to reduced production of Th1 cytokine IFNγ but not IL-2 or IL-4, and reduced production of T cell effector cytokine IL-17. PKC-θ ablation further fails to up-regulate LFA-1 expression in response to TCR activation, which is responsible for T cell transendothelial adhesion, and in some instances LFA-1 upregulation is involved in the induction of EAE. (Tan, et al., "Resistance to experimental autoimmune encephalomyelitis and impaired IL-17 production in protein kinase C {theta}-deficient mice," J. Immunol. 176: 2872-2879 (2006))

PKC-θ is important for the development and persistence of Ag-specific Th1 cells in EAE. A PKC-θ deficiency affects the peripheral T cell responses of mice to MOG, leading to diminished inflammatory cells in CNS tissue and a lowering of Th1 cytokine production, resulting in delayed EAE onset and minimal clinical signs of disease. (Salek-Ardakani, et al., "Protein kinase C{theta} controls Th1 cells in experimental autoimmune encephalomyelitis," J. Immunol. 175: 7635-7641 (2005))

*Inflammatory Bowel Disease*

Inflammatory bowel disease (IBD) is a group of inflammatory conditions of the digestive tract. In some instances, IBD is further classified into Crohn's disease, ulcerative colitis, collagenous colitis, lymphocytic colitis, diversion colitis, Behçet's disease, and indeterminate colitis.

Crohn's disease, also known as Crohn syndrome or regional enteritis, is an IBD that affects the gastrointestinal tract. Symptoms of Crohn's disease include abdominal pain, diarrhea, fever, and weight loss. Additional complications include anemia, skin rashes, arthritis, inflammation of the eye, and tiredness. Although the exact cause is unknown, in some instances, a combination of environmental factors, immune and bacterial factors, and genetic predisposition has been implicated in the development of this disease. In some instances, treatment include antibiotics, 5-aminosalicylic acid (5-ASA) drugs, corticosteroids such as prednisone, immunomodulators such as azathioprine and methotrexate, biologics such as infliximab, adalimumab, certolizumab, and natalizumab, and surgery.

Ulcerative colitis (UC, or Colitis ulcerosa) is a form of IBD that causes inflammation and ulcers in the colon. The symptom of ulcerative colitis include diarrhea which in some instances is mixed with blood and mucus, weight loss, abdominal pain, and anemia. In some instances, treatment include 5-aminosalicylic acid (5-ASA) drugs such as sulfasalazine and mesalazine, corticosteroids such as prednisone, immunosuppressive medications such as azathioprine, and biologics such as infliximab, adalimumab, and golimumab.

*Optic Neuritis*

Optic neuritis is inflammation of the optic neruve. It is further classified into papillitis and retrobulbar neuritis. Papillitis is characterized by inflammation of the optic nerve head, and retrobulbar neuritis is characterized by inflammation of the posterior of the nerve. In some instances, multiple sclerosis is one of the most common etiology of optic neuritis. Additional causes include infection (e.g. syphilis, Lyme disease, herpes zoster), autoimmune disorders (e.g. lupus, neurosarcoidosis, neuromyelitis optica), inflammatory bowel disease, drug induced (e.g. chloramphenicol, ethambutol, isoniazid, streptomycin, quinine, penicillamine, aminosalicylic acid, phenothiazine, phenylbutazone), vasculitis, B12 deficiency and diabetes. The symptoms of optic neuritis include sudden blurred or foggy vision, pain associated with eye movement, impaired color vision, and impaired depth perception. In some instances, treatment includes corticosteroids.

*Neuromyelitis Optica*

Neuromyelitis optica (also known as Devic's disease, Devic's syndrome, or NMO) is a B-cell mediated disease associated with simultaneous inflammation and demyelination of the optic nerve (optic neuritis) and the spinal cord (myelitis). In some instances, the symptoms include vision loss, pain sensation within the eye, sensory disturbances, weakness, numbness and/or paralysis of the arms and legs, and loss of bladder and bowel control. In the disease process, autoantibodies NMO-IgG, derived from peripheral B cells, target CNS astrocytic Aquaporin 4 (AQP4), resulting in complement activation and inflammation. In some instances, the inflammatory lesions are similar to the lesions of multiple sclerosis (MS); however, they differ from MS in their perivascular distribution. There are two variants of neuromyelitis optica, AQP4+ NMO which leads to the attack of astrocytes of the optic nerves and spinal cords by a person's own immune system, and AQP4-NMO, in which the etiology is unknown.

In some embodiments, neuromyelitis optica belongs to a collection of similar diseases termed neuromyelitis optica spectrum disorder (NMOSD). In some cases, the additional diseases belonging to NMOSD comprise Standard Devic's disease, limited forms of Devic's disease, Asian optic-spinal MS, longitudinally extensive myelitis or optic neuritis associated with systemic autoimmune disease, optic neuritis, or NMO-IgG negative NMO.

*Sjögren's Syndrome*

Sjögren's syndrome is a chronic autoimmune disease in which the exocrine glands such as the salivary and lacrimal glands are destroyed by the leukocytes or the white blood cells. In some instances, skin and organs such as kidneys, blood vessels, lungs, liver, biliary system, pancreas, peripheral nervous systems, and the brain are also affected. In some cases, Sjögren's syndrome is classified as primary or secondary Sjögren's syndrome. Symptoms include xerostamia (i.e. dry mouth), keratoconjunctivitis sicca (i.e. dry eyes), joint pain, swollen salivary glands, skin rashes or dry skin, vaginal dryness, persistent dry cough, and prolonged fatigue. In some cases, treatments include parasympathomimetic agonists such as cevimeline and pilocarpine, nonsteroidal anti-inflammatory drugs (NSAIDs), immunosuppressant such as methotrexate, hydroxychloroquine, or surgery.

*Systemic scleroderma*

Systemic scleroderma, also known as systemic sclerosis or SSc, is a connective tissue disease characterized by sclerosis or hardening of skin, blood vessels, and internal organs, and inflammation of joints and muscles. In some instances, systemic scleroderma is further classified into limited cutaneous scleroderma (1cSSc), diffuse cutaneous scleroderma (dcSSc), and systemic sclerosis sine scleroderma (ssSSc). Limited cutaneous scleroderma affects the face, hands and feet, and is characterized by calcinosis, Raynaud phenomenon, esophageal dysfunction, sclerodactyly, and telangiectasia. Diffuse cutaneous scleroderma affects the skins throughout the body and in some instances progress to visceral organs such as the kidneys, heart, lungs and gastrointestinal tract. Systemic sclerosis sine scleroderma is characterized by organ fibrosis in the absence of cutaneous sclerosis. In some cases, treatments include calcium channel blockers, prostanoids, tadalafil, bosentan, corticosteroids, and immunosuppressant.

*Ankylosing Spondylitis*

*Ankylosing* spondylitis (also known as Bekhterev's disease, Marie-Strümpell disease, or AS) is a chronic inflammatory disease of the axial skeleton. *Ankylosing* spondylitis mainly affects the spinal joints and the sacroiliac joint of the pelvis, although in some instances peripheral joints and nonarticular structures are also involved. In some cases, *ankylosing* spondylitis is characterized by the ossification of the outer fibers of the fibrous ring of the intervertebral discs, and in severe cases with complete fusion of the spine. Symptoms of *ankylosing* spondylitis include pain and stiffness of lower back and hips, gradual loss of spinal mobility and chest expansion, limitation of anterior flexion, lateral flexion,

and extension of the lumbar spind. In some instances, treatments include nonsteroidal anti-inflammatory drugs (NSAIDs) such as ibuprofen, phenylbutazone, diclofenac, indomethacin, naproxen and COX-2 inhibitors; opioid analgesics, disease-modifying antirheumatic drugs (DMARDs) such as sulfasalazine; tumor necrosis factor-alpha blockers such as etanercept, infliximab, golimumab, and adalimumab; anti-interleukin-6 inhibitors such as tocilizumab and rituximab.

*Autoimmune hepatitis*

Autoimmune hepatitis (AIH) or lupoid hepatitis is characterized by chronic inflammation of the liver. In some instances, symptoms include fatigue, muscle aches, fever, jaundice, and upper right quadrant abdominal pain. In some cases, autoimmune hepatitis is further classified into four subtypes: positive antinuclear antibody (ANA) and anti-smooth muscle antibody (SMA), characterized by elevated immunoglobulin G; positive liver/kidney microsomal antibody (LKM-1, LKM-2, or LKM-3); positive antibodies against soluble liver antigen; and no autoantibodies detected. In some cases, treatments include glucocorticoids such as budesonide and prednisone; and immunosuppresant such as azathioprine, mycophenolate, cyclosporin, tacrolimus, methotrexate, and the like.

PKC-θ modulates the activation of NKT cells to induce hepatitis. For example, mice deficient in PKC-θ were resistant to concanavalin A (ConA)-induced hepatitis and that ConA-induced production of cytokines such as IFNγ, IL-6, and TNFα, which mediate the inflammation responsible for liver injury, were lower in PKC-θ deficient mice. (Fang, et al., "Ameliorated ConA-induced hepatitis in the absence of PKC-theta," PLoS ONE, 7(2): e31174 (2012))

**Allogeneic Conditions**

*Organ Transplant Rejection*

Organ transplant rejections occur when the transplanted tissue is rejected by the host's immune system. In some instances, the transplanted organs include solid organs such as heart, lungs, kidneys, liver, stomach, pancreas, or intestine, or tissues derived from solid organs such as skin, heart valves, veins, or corneas. In some cases, organ transplant rejection is characterized by hyperacute rejection, acute rejection and chronic rejection. Hyperacute rejection occurs when the transplanted tissue is rejected within minutes or hours due to vascularization damage. Acute rejection occurs within the first six months after transplantation, and further comprises acute cellular rejection and humoral rejection. Chronic rejection occurs after six month of transplantation.

In some instances, alloreactivity in transplantation arises when a mismatch of donor-host human leukocyte antigen (HLA) occurs, leading to subsequent B-cell and T-cell mediated responses. For example, in a B-cell mediated response, allogeneic HLA antigens are internalized by B cells and subsequently processed into peptides for presentation on HLA class-II molecules. Recognition of the HLA class-II presented HLA-derived epitopes by CD4+ T cells results in B-cell activation and IgM to IgG isotype switching. As such, donor-specific IgG HLA alloantibodies are produced which recognize the allogeneic HLA molecules, leading to rejection of the transplanted organ. In a T-cell mediated response, alloreactive T cells either directly recognize intact allogeneic HLA molecules or are involved in indirect recognition by modulating B-cell activation and IgG isotype switching.

In some instances, PKC (e.g., PKC-θ, PKC-α) is involved in survival of activated T cells. Indeed, a study has shown that injection of allogeneic cells into a PKC-θ deficient mice provoked a decreased T cell response compared to WT mice and that alloreactive T cells undergo apoptosis in the absence of PKC-θ. (Sun, Z. "Intervention of PKC-θ as an immunosuppressive regimen," Frontiers in Immunology 3(225): 1-9 (2012); Anderson, et al., "Mice deficient in PKC theta demonstrate impaired in vivo T cell activation and protection from T cell-mediated inflammatory diseases," Autoimmunity 39:469-478 (2006); Manicassamy, et al., "Protein kinase C-{theta}-mediated signals Enhance CD4+ T cell survival by up-regulating Bcl-xL," J. Immunol. 176:6709-6716 (2006)) A second study shows that a combination of PKC-θ/PKC-α deficiency leads to an additive T cell response defects (Gruber, et al., "PKCθ cooperates with PKCα in alloimmune responses of T cells in vivo," Molecular Immunology 46:2071-2079 (2009)).

In some embodiments, there are several different treatment options for acute rejection. Exemplary treatment options include corticosteroids such as prednisolone and hydrocortisone; calcineurin inhibitors such as cyclosporin and tacrolimus; anti-proliferatives such as azathioprine and mycophenolic acid; mTOR inhibitors such as sirolimus and everolimus; biologics such as monoclonal anti-IL-2Rα receptor antibodies (e.g., basiliximab, daclizumab), polyclonal anti-T-cell antibodies (e.g., anti-thymocyte globulin and antilymphocyte globulin), and monoclonal anti-CD20 antibodies (e.g., rituximab). For hyperacute rejection, the sole treatment option is removal of the tissue, and for chronic rejection, retransplant is proposed as the preferred option.

PKC-θ enhances T cell survival and promotes the differentiation of naive T cells into inflammatory Th17 cells. Furthermore, modulation of PKC-θ activity shifts the ratio between inflammatory effector T cells and inhibitory Tregs, to control T cell-mediated immune responses that are responsible for autoimmunity and allograft rejection. Indeed, PKC-θ-deficient mice are resistant to the development of several Th2 and Th17-dependent autoimmune diseases and are

defective in mounting alloimmune responses required for rejection of transplanted allografts and graft-versus-host disease. (Sun, Z. "Intervention of PKC-θ as an immunosuppressive regiment," Frontiers in Immunology, 3(225):1-9 (2012))

*Graft vs Host Disease*

Graft vs host disease (GvHD) is a complication following an allogeneic stem cell transplant, and is characterized by a T cell-mediated recognition of minor histocompatibility antigens followed by organ-specific vascular proliferation, cytokine release, and direct cell-mediated attack on normal tissues. In some cases, the stem cells are obtained from bone marrow, peripheral blood, or cord blood. In some instances, there are two types of GvHD, acute or fulminant form of GvHD (aGvHD), and chronic form of GvHD (cGvHD). Acute GvHD occurs within the first 100 days of transplant while chronic GvHD occurs after the 100 day time frame. In some instances, treatment of GvHD include calcineurin inhibitors such as cyclosporine and tacrolimus; mTOR inhibitors such as sirolimus; and antiproliferative agents such as methotrexate, cyclophosphamide, and mycophenolate.

PKC-θ plays an important role in lowering the overall signaling threshold required for T cell activation. Therefore, the absence of PKC-θ selectively impairs T cell activation by low-level and low-affinity TCR agonists. As such, in an allogeneic setting, inhibition of PKC-θ can prevent GVHD induction while maintaining the ability to respond to virus infection and to induce graft-versus-leukemia (GVL) effect after BM transplantation. (Valenzuela, et al., "PKCθ is required for alloreactivity and GVHD but not for immune responses toward leukemia and infection in mice," The Journal of Clinical Investigation, 119(12): 3774-3786 (2009))

**Methods of Treatment**

*Rheumatoid arthritis*

Also described herein is a method of treating rheumatoid arthritis in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient. In one embodiment, the rheumatoid arthritis is rheumatoid factor positive (seropositive) RA, rheumatoid factor negative (seronegative) RA, and juvenile RA (or juvenile idiopathic arthritis).

*Multiple Sclerosis*

Also described herein is a method of treating multiple sclerosis in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient. In one embodiment, the multiple sclerosis is relapsing-remitting (RR) MS, secondary progressive (SP) MS, primary progressive (PP) MS, progressive relapsing MS, clinically isolated syndrome (CIS), or radiologically isolated syndrome (RIS).

*Inflammatory Bowel Disease*

Also described herein is a method of treating inflammatory bowel disease (IBD) in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient. In one embodiment, the IBD is Crohn’s disease, ulcerative colitis, collagenous colitis, lymphocytic colitis, diversion colitis, Behçet’s disease, or indeterminate colitis.

Also described herein is a method of treating Crohn’s disease in a subject in need thereof comprising administering to the subject a compound of having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine.

Also described herein is a method of treating ulcerative colitis comprising administering to the subject a compound of having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine.

*Optic Neuritis*

Also described herein is a method of treating optic neuritis in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient. In one embodiment, the optic neuritis is papillitis neuritis or retrobulbar neuritis.

*Neuromyelitis Optica*

Also described herein is a method of treating neuromyelitis optica (NMO) in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

*Sjögren's Syndrome*

Also described herein is a method of treating Sjögren's syndrome in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

*Systemic Scleroderma*

Also described herein is a method of treating systemic scleroderma in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient. In one embodiment, the systemic scleroderma is limited cutaneous scleroderma (lcSSc), diffuse cutaneous scleroderma (dcSSc), or systemic sclerosis sine scleroderma (ssSSc).

*Ankylosing Spondylitis*

Also described herein is a method of treating ankylosing spondylitis in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

*Autoimmune Hepatitis*

Also described herein is a method of treating autoimmune hepatitis in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

*Graft vs Host Disease*

Also described herein is a method of treating GVHD in a subject in need thereof comprising administering to the subject a composition comprising a compound having the formula 5-{ [(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient. In one embodiment, the GVHD is acute GVHD or chronic GVHD

*Organ Transplant Rejection*

Also described herein is a method of treating organ transplant rejection in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

*Autoimmune or Alloimmune diseases*

Also described herein is a method of treating an autoimmune or alloimmune disease selected from rheumatoid arthritis, multiple sclerosis, inflammatory bowel disease (IBD) (e.g., Crohn's disease, ulcerative colitis), optic neuritis, Neuromyelitis Optica, Sjögren's syndrome, systemic scleroderma, Ankylosing Spondylitis, autoimmune hepatitis, Graft vs host disease (GvHD), and organ transplant rejection in a subject in need thereof comprising administering to the subject a composition comprising a compound having the formula 5-{[(2S,5R)-2,5-dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof, and a pharmaceutically acceptable excipient.

*Pharmaceutical compositions and dosage forms*

The pyrrolo-pyrazole compounds used in the methods described herein are, in some instances, administered orally as tablets or capsules, as oily or aqueous suspensions, lozenges, troches, powders, granules, emulsions, syrups or elixirs. The compositions for oral use may include one or more agents for flavoring, sweetening, coloring and preserving in order to produce pharmaceutically elegant and palatable preparations. Tablets may contain pharmaceutically acceptable excipients as an aid in the manufacture of such tablets. As is conventional in the art these tablets may be coated with a pharmaceutically acceptable enteric coating, such as glyceryl monostearate or glyceryl distearate, to delay disintegration and absorption in the gastrointestinal tract to provide a sustained action over a longer period.

Formulations for oral use may be in the form of hard gelatin capsules wherein the active ingredient is mixed with an inert solid diluent, for example, calcium carbonate, calcium phosphate or kaolin. They may also be in the form of soft gelatin capsules wherein the active ingredient is mixed with water or an oil medium, such as peanut oil, liquid paraffin or olive oil.

Aqueous suspensions normally contain active ingredients in admixture with excipients suitable for the manufacture of an aqueous suspension. Such excipients may be a suspending agent, such as sodium carboxymethyl cellulose, methyl cellulose, hydroxypropylmethyl cellulose, sodium alginate, polyvinylpyrrolidone, gum tragacanth and gum acacia; a dispersing or wetting agent that may be a naturally occurring phosphatide such as lecithin, a condensation product of ethylene oxide and a long chain fatty acid, for example polyoxyethylene stearate, a condensation product of ethylene oxide and a long chain aliphatic alcohol such as heptadecaethylenoxycetanol, a condensation product of ethylene oxide and a partial ester derived from a fatty acid and hexitol such as polyoxyethylene sorbitol monooleate or a fatty acid hexitol anhydrides such as polyoxyethylene sorbitan monooleate.

The pyrrolo-pyrazole compounds used in the methods described herein are, in some instances, in the form of a sterile injectable aqueous or oleagenous suspension. This suspension may be formulated according to know methods using those suitable dispersing or wetting agents and suspending agents that have been mentioned above. The sterile injectable preparation may also be formulated as a suspension in a non toxic perenterally-acceptable diluent or solvent, for example as a solution in 1,3-butanediol. Among the acceptable vehicles and solvents that may be employed are water, Ringers solution and isotonic sodium chloride solution. For this purpose any bland fixed oil may be employed including synthetic mono- or diglycerides. In addition fatty acids such as oleic acid find use in the preparation of injectables.

Dosage levels of the pyrrolo-pyrazole compounds to be used for the methods of treatment disclosed herein range from about 0.5 mg/kg body weight to about 100 mg/kg body weight. A preferred dosage range is between about 30 mg/kg body weight to about 100 mg/kg body weight.

**EXAMPLES**

These examples are provided for illustrative purposes only.

Compound A refers to 5-{[(2S,5R)-2,5-Dimethyl-4-(tetrahydro-2H-pyran-4-ylmethyl)piperazin-1-yl]carbonyl} -N-(5-fluoro-2-methylpyrimidin-4-yl)-6,6-dimethyl-1,4,5,6-tetrahydropyrrolo[3,4-c]pyrazol-3-amine, which was disclosed in WO 2008/096260 and having the chemical structure:

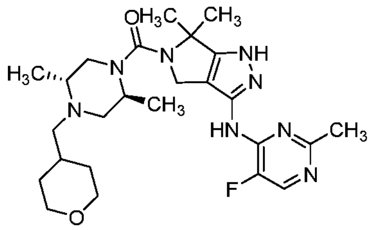

A summary of PKC inhibition by compound A is provided in Table 1. The methods for these determinations have been described (Grant, et al. 2010, Eur J Pharmacol . 627:16-25). Compound A is a potent, ATP-competitive and reversible inhibitor of conventional PKC enzymes with a Ki = 5.3 nM for recombinant PKC beta and a Ki = 10.4 nM for recombinant PKC alpha. It also is a potent inhibitor of the novel isoform PKC theta with an ICso = 25.6 nM. Furthermore, it demonstrated some potency for conventional isoform PKC gamma with an ICso = 57.5 nM. Otherwise, it demonstrated a high degree of selectivity for the other members of the conventional, novel and atypical isoforms of PKC as shown by lower potency against these isoforms (Table 1). Compound A does not significantly inhibit PKC delta.

**Table 1**

| In Vitro Assays | $IC_{50}$ (nM) | Ki (nM) |
|---|---|---|
| Human PKC alpha | | 10.4 |
| Human PKC betaII | | 5.3 |
| Human PKC alpha | 2.3 | |
| Human PKC betaI | 8.1 | |
| Human PKC betaII | 7.6 | |
| Human PKC theta | 25.6 | |
| Human PKC gamma | 57.5 | |
| Human PKC mu | 314 | |
| Human PKC epsilon | 808 | |
| Human PKC delta | > 1000 | |
| Human PKC eta | > 1000 | |
| Human PKC iota | > 1000 | |
| Human PKC zeta | > 1000 | |
| Human PRKCN (PKD3) | 131 | |
| pSHP2 (PKCβ cell assay) | 9.8 | |
| Interleukin-8 release | 39 | |

**Example 1: Testing in type II collagen arthritis mouse model of rheumatoid arthritis**

This study was carried out to determine the potential efficacy of compound A in inhibiting the inflammation, cartilage destruction, pannus formation and bone resorption associated with type II collagen arthritis in mice.

*Test System*

Male DBA/1 OlaHsd mice (*N*= 56) that were 6-7 weeks old upon arrival were obtained from Harlan, Inc., Indianapolis, Indiana. Mice weighed approximately 15-24 grams (mean 21 g) at enrollment on Study Day 17.

Animals were acclimated for at least 4 days after arrival at BBP. They were identified by a distinct number of ink marks at the base of the tail delineating animal number. After enrollment, all cages were labeled with protocol number, group number, and animal numbers.

*Collagen Preparation*

Equal volumes of a 4 mg/ml Type II collagen solution (in 0.01N Acetic acid) and a 5 mg/ml Freund's complete adjuvant (1 mg/ml FCA supplemented with heat killed mycobacterium tuberculosis) were emulsified by hand mixing with syringes for approximately 5 min, at which point a bead of this material holds its form when placed in water. The final concentrations were 2 mg/ml of Type II Collagen and 2.5 mg/ml of Freund's complete adjuvant.

*Experimental Design*

The basic study design and animal usage was approved by Bolder BioPATH's Institutional Animal Care and Use Committee (IACUC) for compliance with regulations prior to study initiation (IACUC Protocol #BBP-001).

Mice were anaesthetized with Isoflurane, shaved at the base of the tail, and injected intradermally with 100 μl of Freund's Complete Adjuvant (Sigma Aldrich, cat #R134067) containing bovine type II collagen (BBP Batch #8) (1 mg/ml) at the base of the tail on day 0 and again on day 21. On Study Day 17, mice were weighed and randomized into treatment groups. Treatment was initiated on Day 18 and continued through Study Day 35 (Table 1). Arthritis occurred on Study Days 24-35. Mice were terminated on Day 35. Clinical scores were given for each of the paws (right front, left front, right rear, left rear) on Study Days 18-35.

Dose formulations for each dose level were prepared once within 24 hours prior to the administration of the first dose. The dose formulations were stored in a 2 °C-8 °C fridge and protected from light when not in use. The amount needed for each am and pm dosing was removed just prior to the time of dosing while the remaining dosing solution was kept in the refrigerator. A new batch of dosing solution was prepared every 7-10 days for the duration of the experiment.

Vehicle was prepared as a 0.5% methylcellulose (400 cps) solution in purified water.

Dexamethasone was prepared as a 2mg/ml stock. The 0.2 mg/kg solution was prepared by diluting 0.255 mls of the stock solution with 50.745 mL of 1% carboxymethylcellulose (BBP Batch 2015).

Compound A at 60, 90, and 120 mg/kg concentrations were prepared containing 0.5% (w/v) methylcellulose in purified water. The dosing concentration of compound A was calculated based on the correction factor of 1.04.

**Table 1. Group and Treatment Information**

| Group | N | Disease | Treatment | Dose Level (mg/kg) | Dose Route | Regimen[1] | Dosing Days | Dose Vol. (ml/kg)[2] |
|---|---|---|---|---|---|---|---|---|
| 1 | 4 | N | Normal | N/A | PO | BID | 18-35 | 10 |
| 2 | 12 | Y | Vehicle Control | N/A | PO | BID | 18-35 | 10 |
| 3 | 4 | Y | Dexamethasone | 0.1 | PO | BID | 18-35 | 10 |
| 4 | 12 | Y | Compound A | 60 mg/kg | PO | BID | 18-35 | 10 |
| 5 | 12 | Y | Compound A | 90 mg/kg | PO | BID | 18-35 | 10 |
| 6 | 12 | Y | Compound A | 120 mg/kg | PO | BID | 18-35 | 10 |

[1]BID dosing occurred at approximately 10-12 h intervals.
[2]The doses of compound A were calculated daily in mg/kg based on the latest body weight of the animal.

Mice were weighed on Study Days 17, 20, 22, 24, 26, 28, 30, 32, 34, and 35.

Daily clinical scores were given for each of the paws (right front, left front, right rear, left rear) on Study Days 18-35 using the following criteria:

0 = Normal.
1 = One hind or fore paw joint affected or minimal diffuse erythema and swelling.
2 = Two hind or fore paw joints affected or mild diffuse erythema and swelling.
3 = Three hind or fore paw joints affected or moderate diffuse erythema and swelling.
4 = Four hind or fore paw joints affected or marked diffuse erythema and swelling.
5 = Entire paw affected, severe diffuse erythema and severe swelling, unable to flex digits.

On Study Day 35, mice were anesthetized with Isoflurane (VetOne, cat #502017) and bled by cardiac puncture for plasma (K2EDTA) and serum. Plasma and serum samples were stored frozen at -80°C.

Also on Study Day 35, after terminal bleeds, animals were euthanized by cervical dislocation. Fore paws, hind paws, and knees were harvested and placed in 10% neutral buffered saline (NBF) for microscopy.

Animal carcasses were disposed of according to Bolder BioPATH, Inc. procedures.

*Statistical Analysis*

Clinical data for paw scores (means for animal) were analyzed by determining the area under the dosing curve (AUC) for Study Days 18-35. For calculation of AUC, the daily mean scores for each mouse were entered into Microsoft

Excel and the area between the treatment days and the final day was computed. Means for each group were determined. A one-way analysis of variance (1-way ANOVA) or Kruskal-Wallis test (non-parametric), along with the appropriate multiple comparison post-test (Dunnett's or Dunn's), was used to evaluate data collected in this study. A Student's two-tailed t-test was used to compare normal versus disease controls for model validation. Unless indicated, Bolder BioPATH, Inc. performs statistical analysis on raw (untransformed) data only. Statistical tests make certain assumptions regarding normality and homogeneity of variance, and further analysis may be required if testing resulted in violations of these assumptions. P values were rounded to three decimal places. Significance for all tests was set at $p < 0.050$. Statistical analysis was performed using Prism 6.0d software (GraphPad). Percent inhibition is calculated using the following formula:

$$\% \text{ Change} = B/A \text{ X } 100$$

A = Mean Normal - Mean Disease Control
B = Mean Treated - Mean Disease Control

*Life Phase and Necropsy Parameters*

Vehicle-treated mice had body weight loss (measured as percent change from baseline) that began at the onset of arthritis and peaked at -11.66% on Study Day 34. Disease-induced body weight loss was inhibited on Days 20 and 34 in mice treated with 120 mg/kg compound A compared to Vehicle Controls. Results of treatment with compound A were dose responsive. Body weight loss was increased on Day 20 in mice treated with Dex compared to Vehicle Controls. Vehicle Control mice had absolute body weight loss (Day 17-35) of -1.68 g. Absolute body weight loss was not affected by treatment (Figures 1-3, Table 2).

Arthritis scores measured daily differed from Vehicle Controls over time. Clinical arthritis scores were reduced toward normal in mice treated with 120 mg/kg compound A (*$p < 0.05$ on Days 27-35) or Dex (*D29-35) compared to Vehicle Controls. Arthritis scores expressed as area under the curve (AUC) were reduced in mice treated with 120 mg/kg compound A (72% reduction) or Dex (100%) compared to Vehicle Controls. Results of treatment with compound A were dose responsive (Figures 4-5, Table 2).

Absolute body weight loss was not affected. Vehicle Control mice had 100% disease incidence by Study Day 30. Disease incidence was reduced in mice treated with 90 or 120 mg/kg compound A (92% and 83% incidence at study termination, respectively) or Dex (0%) (Figure 6, Table 1, Table 2).

Dexamethasone, a steroid medication with anti-inflammatory effects, was used as a positive control for this study. Treatment with dexamethasone (0.1 mg/kg) showed an effect in inhibiting clinical arthritis scores AUC when compared to Vehicle Control mice. In addition, Vehicle Control mice displayed an increase in clinical arthritis scores AUC when compared to naive (age matched, non-diseased) mice.

**Table 2. Summary of Clinical Data**

| Group | Treatment | Change in Body Weight from Day 17 (g) | Paw Score AUC | Percent Incidence |
|---|---|---|---|---|
| 1 | Normal (vehicle) PO, BID | †0.33 (0.14) | †0.00 (0.00) | 0% |
| 2 | Vehicle Control PO, BID | -1.68 (0.38) | 20.27 (3.14) | 100% |
| 3 | Dexamethasone (0.1 mg/kg) PO, BID | -2.44 (0.68) | *0.00 (0.00) | 0% |
| 4 | compound A (60 mg/kg) PO, BID | -1.55 (0.48) | 22.91 (2.82) | 100% |
| 5 | compound A (90 mg/kg) PO, BID | -0.86 (0.52) | 9.85 (2.37) | 92% |
| 6 | compound A (120 mg/kg) PO, BID | -0.25 (0.26) | *5.74 (1.41) | 83% |
| (SE) = Standard error displayed in parenthesis, AUC = Area Under the Curve<br>Vehicle = (0.5% methylcellulose (400 cps) in purified water)<br>*$p < 0.05$ ANOVA (with Dunnett's post-hoc test) or K-W test (with Dunn's post-hoc test) vs. Vehicle Control<br>†$p < 0.05$ Student's *t*-test to Vehicle (comparison to Naive) | | | | |

**Example 2: A double-blind, proof-of-concept safety and activity study of Compound A versus placebo as co-therapy with methotrexate in active rheumatoid arthritis**

| | |
|---|---|
| Study design: | Allocation: randomized |
| Endpoint Classification: | Safety/Efficacy Study |
| Intervention Model: | Parallel Assignment |
| Masking: | Double Blind (Subject, Caregiver, Investigator, Outcomes Assessor) |
| Primary Purpose: | Treatment |

*Primary Outcome Measures:*

American College of Rheumatology Criteria for a 20% improvement (ACR 20) [ Time Frame: Week 4 ] [ Designated as safety issue: No ]

Percentage of participants with an American College of Rheumatology ≥20% (ACR20) response. A participant is a responder if the following 3 criteria for improvement from Baseline are met: ≥ 20% improvement in 68 tender joint count; ≥ 20% improvement in 66 swollen joint count; and ≥ 20% improvement in at least 3 of the 5 following parameters: Patient's assessment of pain (measured on a 100 mm visual analog scale [VAS]); Patient's global assessment of disease activity (measured on a 100 mm VAS); Physician's global assessment of disease activity (measured on a 100 mm VAS); Patient's self-assessment of physical function (Health Assessment Questionnaire - Disability Index (HAQ-DI)); C-Reactive Protein.

*Secondary Outcome Measures:*

Number of participants with adverse events [ Time Frame: Up to 8 Weeks ] [ Designated as safety issue: Yes ]

Safety and tolerability of Compound A compared with placebo in subjects on a background of stable MTX therapy. An adverse event (AE) is any noxious, unintended, or untoward medical occurrence that may appear or worsen in a subject during the course of a study. In some cases, it is a new intercurrent illness, a worsening concomitant illness, an injury, or any concomitant impairment of the subject's health, including laboratory test values (as specified by the criteria below), regardless of etiology. Any worsening (ie, any clinically significant adverse change in the frequency or intensity of a preexisting condition) should be considered an AE.

American College of Rheumatology Criteria for a 50% improvement (ACR 50) [ Time Frame: Week 4 ] [ Designated as safety issue: No ]

Percentage of participants with an American College of Rheumatology ≥50% (ACR50) response. A participant is a responder if the following 3 criteria for improvement from Baseline are met:

a. ≥ 50% improvement in 68 tender joint count;
b. ≥ 50% improvement in 66 swollen joint count; and
c. ≥ 50% improvement in at least 3 of the 5 following parameters:

   i. Patient's assessment of pain (measured on a 100 mm visual analog scale [VAS]);
   ii. Patient's global assessment of disease activity (measured on a 100 mm VAS);
   iii. Physician's global assessment of disease activity (measured on a 100 mm VAS);
   iv. Patient's self-assessment of physical function (Health Assessment Questionnaire - Disability Index (HAQ-DI));
   v. C-Reactive Protein.

American College of Rheumatology Criteria for a 70% improvement (ACR 70) [ Time Frame: Week 4 ] [ Designated as safety issue: No ]

Percentage of participants with an American College of Rheumatology ≥70% (ACR70) response. A participant is a responder if the following 3 criteria for improvement from Baseline are met:

a. ≥ 70% improvement in 68 tender joint count;
b. ≥ 70% improvement in 66 swollen joint count; and
c. ≥ 70% improvement in at least 3 of the 5 following parameters:

   i. Patient's assessment of pain (measured on a 100 mm visual analog scale [VAS]);

ii. Patient's global assessment of disease activity (measured on a 100 mm VAS);
iii. Physician's global assessment of disease activity (measured on a 100 mm VAS);
iv. Patient's self-assessment of physical function (Health Assessment Questionnaire - Disability Index (HAQ-DI));
v. C-Reactive Protein.

*Eligibility*

| | |
|---|---|
| Ages Eligible for Study: | 18 Years to 80 Years |
| Genders Eligible for Study: | Female |
| Accepts Healthy Volunteers: | No |

*Inclusion Criteria:*

Female ≥18 years of age at the time of signing the informed consent.

Must meet the 2010 ACR/EULAR Classification Criteria for RA, have RA for at least 6 months, and must continue to have active RA at the time of randomization despite at least 3 months of treatment with stable doses of MTX (7.5 to 25 mg/week oral or parenteral) for at least 4 weeks prior to randomization.

Must have been treated with MTX for at least 3 months prior to randomization, and must be on a stable dose between 7.5 and 25 mg/week (PO or parenteral, not both) for at least 4 weeks prior to randomization. Subjects will be required to maintain their stable dose through Day 28/Week 4 of the study. Oral folate supplementation is required with a minimum dose of 5 mg/week (ie, folic acid) while the subject is taking MTX. Leucovorin may be used instead of folic acid and may be dosed up to 10 mg/week orally.

Sulfasalazine is allowed as a concomitant medication, however, subject must be on a stable dose for at least 4 weeks prior to randomization and through Day 28/Week 4 of the study.

Hydroxychloroquine or chloroquine is allowed as concomitant medications, however, subject must be on a stable dose for at least 4 weeks prior to randomization and through Day 28/Week 4 of the study.

Modification of Diet in Renal Disease formula (MDRD) estimated glomerular filtration rate (MDRD eGFR) ≥ 60 mL/min/1.73m2+

*Exclusion Criteria:*

Currently using treatment with DMARDs (other than sulfasalazine, hydroxychloroquine or chloroquine and MTX), including biologics. Previous use is only allowed after adequate washout (4 weeks or 5 half-lives, whichever is longer) prior to randomization.

Previous treatment with any cell depleting therapies, including investigational agents (eg, CAMPATH, anti-CD4, anti-CD5, anti-CD3, anti-CD19 and anti-CD20) within 6 months of screening.

Treatment with intravenous gamma globulin, plasmapheresis or Prosorba® column within 2 weeks prior to randomization.

Intra-articular or parenteral corticosteroids are not allowed within 2 weeks prior to randomization.

**Example 3: A phase II double-blind, placebo-controlled, multiple dose study to evaluate different doses of Compound A in rheumatoid arthritis patients failing to respond to methotrexate**

| | |
|---|---|
| Study design: | Allocation: randomized |
| Endpoint Classification: | Safety/Efficacy Study |
| Intervention Model: | Parallel Assignment |
| Masking: | Double Blind (Subject, Caregiver, Investigator, Outcomes Assessor) |
| Primary Purpose: | Treatment |

*Primary Outcome Measures:*

The Primary Efficacy parameter is ACR20 response rate at 3 months post dosing. [ Time Frame: 12 weeks ]

[ Designated as safety issue: No ]

*Secondary Outcome Measures:*

ACR 20/50 responses over time [ Time Frame: 12 weeks ]
[ Designated as safety issue: No ]

Disease Activity Score (DAS) at baseline and endpoint [ Time Frame: 12 Weeks ] [ Designated as safety issue: No ]

Mean changes (SDs) from baseline in Swollen Joint Count (28 joint count) [ Time Frame: 12 Weeks ] [ Designated as safety issue: No ]

Mean changes (SDs) from baseline in Tender Joint Count (28 joint count) [ Time Frame: 12 Weeks ] [ Designated as safety issue: No ]

Mean changes (SDs) from baseline in Physician global assessment of disease activity by visual analog scale (VAS) [ Time Frame: 12 Weeks ] [ Designated as safety issue: No ]

Mean changes (SDs) from baseline in Patient global assessment of disease activity by VAS [ Time Frame: 12 Weeks ] [ Designated as safety issue: No ]

Mean changes (SDs) from baseline in Patient assessment of pain by VAS [ Time Frame: 12 Weeks ] [ Designated as safety issue: No ]

Mean changes (SDs) from baseline in HAQ-DI [ Time Frame: 12 Weeks ] [ Designated as safety issue: No ]

Mean changes (SDs) from baseline in CRP [ Time Frame: 12 Weeks ] [ Designated as safety issue: No ]

The frequency and severity of Liver Function Test abnormalities, especially ALT and alkaline phosphatase [ Time Frame: 12 Weeks ] [ Designated as safety issue: Yes ]

The frequency and severity of hematopoietic cytopenias, principally effects on neutrophil, erythrocyte, and lymphocyte counts [ Time Frame: 12 Weeks ]
[ Designated as safety issue: Yes ]

The frequency and severity of clinically significant adverse events, especially skin rash, postural dizziness, and alterations in blood pressure and other relevant clinical outcomes [ Time Frame: 12 Weeks ] [ Designated as safety issue: Yes ]

Eligibility

| | |
|---|---|
| Ages Eligible for Study: | 18 Years to 75 Years |
| Genders Eligible for Study: ] | Both |
| Accepts Healthy Volunteers: | No |

*Inclusion Criteria:*

Males and females, 18 to 75 years of age, with active RA for at least 12 months (functional class I-III, e.g., not bed or wheelchair-bound) who have been receiving weekly doses of methotrexate (10-25 mg/week) for a minimum of 180 days, and who have been receiving a stable MTX dose of at least 15 mg without any change in route or change in folic acid supplementation for at least 30 days.

Active RA is defined as the presence of (a) 6 swollen joints (28 joint count); AND (b) 6 tender joints (28 joint count); AND (c) CRP level > ULN for the central reference laboratory. Patient may receive up to 10 mg per day of oral prednisone or steroid equivalent, NSAID therapy, hydroxychloroquine, chloroquine, minocycline, sulfasalazine, and doxycycline. The dose(s) must have been stable for at least 30 days and must not be changed during the washout, screening and treatment periods, unless dictated by tolerability requirements.

The patient is in otherwise good health as determined by the Investigator on the basis of medical history, physical examination, and laboratory screening tests during the screening period, including the absence of clinically significant findings, such as HIV, HBV or HCV, interstitial pneumonitis or active pulmonary infection, on chest X-ray taken within 6 months prior to screening and a negative TB skin test, or abnormal liver function defined as known ALT >1.2xULN within the past 90 days.

In the investigator's opinion, the patient has the ability to understand the nature of the study and any hazards of participation, and to communicate satisfactorily with the investigator and to participate in, and to comply with, the requirements of the entire protocol.

*Exclusion Criteria:*

The patient has a history of, or a concurrent, clinically significant illness, medical condition (other than arthritis)

or laboratory abnormality that, in the Investigator's opinion, could affect the conduct of the study (these will be included in an exclusion log).

The patient has a history of substance abuse, drug addiction or alcoholism.

The patient is unable to abstain from alcohol during the study.

The patient has received any investigational medication within 30 days prior to admission to the study.

Any patient who has received any of the following treatments must abide by the indicated washout period:

a. oral or injectable gold, azathioprine, penicillamine, anakinra require a 30 day washout period prior to Day 1 dosing
b. cyclosporine, abatacept, etanercept, infliximab or adalimumab require a 60 day washout period prior to Day 1 dosing
c. leflunomide requires a 60 day washout period prior to screening, unless the patient has undergone cholestyramine washout at least 30 days prior to Day 1 dosing
d. cyclophosphamide requires a 180 day washout period prior to Day 1 dosing
e. Rituxan requires a 180 day washout period and normal CD19 count prior to Day 1 dosing
f. parenteral or intra-articular corticosteroids require a 30 day washout period prior to Day 1 dosing

Patients with the following laboratory abnormalities: ALT > 1.2X ULN, creatinine > ULN, a neutrophil count < 2,500/mm3 or lymphocyte count < 800/mm3, Hgb < 10 g/dL, platelet count < 125,000/mm3 are excluded.

**REFERENCE Example 4: Evaluate the efficacy and safety of Compound A in subjects with Psoriasis**

A 2-week multiple ascending dose, double-blind placebo-controlled study to evaluate the safety, tolerability, and pharmacokinetics of twice daily administration of oral Compound A and to explore the pharmacodynamics of oral Compound A in moderate to severe psoriasis patients.

Patients aged between 18 and 65 years with stable plaque psoriasis and no other clinically significant abnormalities are enrolled. Patients are hospitalized for study weeks 1 and 2 and are further closely monitored as outpatients on a weekly basis during study weeks 3 and 4. Four consecutive cohorts of 8 patients each are treated with oral Compound A for 2 weeks with rising doses (6 patients in each cohort) or with placebo (2 patients in each cohort). Escalating doses of Compound A are administered. The start of the next higher dose level is permitted only after demonstration of tolerability and safety at the preceding lower dose. Blood pressure, pulse rate, ECG evaluations, and hematology/blood chemistry laboratory parameters are closely monitored from day -1 and throughout the study period, including the follow-up period at days 21 and 28. Creatinine clearance is determined at days -1 and 14. Disease severity is assessed on a weekly basis using PASI, a validated score widely used in clinical research in psoriasis. Skin biopsies (5 mm) are taken from typical psoriatic plaques from all patients at baseline (day 0), day 7, and day 14 (end of treatment period). Epidermal thickening and the enumeration of $CD3^+$, $CD14^+$, $CD15^+$, $CD207^+$, and Ki-$67^+$ cells together with a K16 evaluation are analyzed as described by Bangert, et al., (Immunopathologic features of allergic contact dermatitis in humans: participation of plasmacytoid dendritic cells in the pathogenesis of the disease? J. Invest. Dermatol. 121:1409-1418 (2003)).

**REFERENCE Example 5: A double blind, randomized, placebo controlled study of Compound A assessing psoriasis area and severity index (PASI) response in patients with psoriasis**

| | |
|---|---|
| Study design: | Allocation: randomized |
| Intervention Model: | Parallel Assignment |
| Masking: | Double Blind (Subject, Investigator) |
| Primary Purpose: | Treatment |

*Primary Outcome Measures:*

Change from baseline in plaque psoriasis as assessed by PASI response or PASI 75 (a patient that has an improvement from baseline PASI of at least 75%) [ Time Frame: to 12 weeks treatment ] [ Designated as safety issue: No ]

*Secondary Outcome Measures:*

ECG and laboratory parameters, rates of AEs, and percentage of patients requiring interruption or discontinuation of study drug due to AEs [ Time Frame: up to 12 weeks treatment ] [ Designated as safety issue: Yes ]

Change in PASI and Investigator's Global Assessment (IGA) of psoriasis in patients receiving compound A compared with placebo [ Time Frame: up to 12 weeks treatment ] [ Designated as safety issue: No ]
Disease recurrence (PASI, IGA) and the effect of treatment withdrawal (including AEs) in the treatment-free Follow-up Period [ Time Frame: in the treatment-free Follow-up Period ] [ Designated as safety issue: No ]

*Eligibility*

| | |
|---|---|
| Ages Eligible for Study: | 18 Years to 75 Years |
| Genders Eligible for Study: | Both |
| Accepts Healthy Volunteers: | No |

*Inclusion Criteria:*

Moderate and Severe Plaque psoriasis diagnosed for at least 12 months (with or without psoriatic arthritis as comorbidity)that requires systemic therapy
Severity of disease meeting all of the following three criteria:

a. PASI score of 10 or greater
b. Total Body Surface Area (BSA) affected by plaque psoriasis of 10% or greater
c. Investigator's Global Assessment (IGA) score of 3 or greater

*Exclusion Criteria:*

- Hematological abnormalities
- Heart rate < 50 or > 90 bpm when resting for 5 minutes
- Family history of long QT syndrome
- History of tachyarrhythmia
- History of conduction abnormality i.e., PR > 200 msec, 2nd or 3rd degree AV block, complete left or right branch bundle block, pre-excitation syndrome
- Uncontrolled or unstable angina pectoris; history of myocardial infarction within the previous 12 months
- Known history of congestive heart failure
- History of percutaneous coronary intervention (PCI) or cardiac ablation
- History of stroke or transient ischemic attack (TIA)
- Implanted cardiac pacemaker or defibrillator
- History of malignancy of any organ system
- Current guttate, generalized erythrodermic, or pustular psoriasis
- Current drug associated psoriasis

**Example 6: Testing in Tight skin-1 (TSK-1) mouse model of Systemic Scleroderma**

Five-week-old male and female tight skin-1 (TSK-1) mice are used in the study. The animals are randomized by body weight into one of the treatment groups or an untreated control group. Treatments are initiated after randomization and continue for 10 weeks. At the end of 10 weeks, mice are sacrificed. Lesional skin areas are excised, fixed in 4% formalin, and embedded in paraffin. The hypodermal thickness in TSK-1 mice is determined by measuring the thickness of the subcutaneous connective tissue beneath the panniculus carnosus at four different sites of the upper back in each mouse.
The collagen content in lesional skin samples is evaluated by the hydroxyproline assay. After digestion of punch biopsy specimens (3 mm diameter) in 6 M HCl for 3 hours at 120°C, the pH of the samples is adjusted to 7. Afterwards, samples are mixed with 0.06 M chloramine T and are incubated for 20 minutes at room temperature. Then, 3.15 M perchloric acid and 20% p-dimethylaminobenzaldehyde are added, and samples are incubated for an additional 20 minutes at 60°C. The absorbance is determined at 557 nm with a spectrophotometer. For direct visualization of collagen fibers, trichrome staining is performed.

**Example 7: A 24-week, proof of concept study to evaluate the efficacy and tolerability of Compound A for the treatment of fibrosis in patients with systemic sclerosis**

| | |
|---|---|
| Study design: | Endpoint Classification: Safety/Efficacy Study |
| Intervention Model: | Single Group Assignment |
| Masking: | Open Label |
| Primary Purpose: | Treatment |

*Primary Outcome Measures:*

Percentage change from baseline in modified Rodnan Skin Score (MRSS) at each time point of analysis [ Time Frame: 24 weeks ] [ Designated as safety issue: No ]

*Secondary Outcome Measures:*

Assessment of non-response, partial response, complete response, remission or relapse assessed by MRSS values. [ Time Frame: 48 weeks ] [ Designated as safety issue: No ]

*Eligibility*

| | |
|---|---|
| Ages Eligible for Study: | 18 Years and older |
| Genders Eligible for Study: | Both |
| Accepts Healthy Volunteers: | No |

*Inclusion Criteria:*

Male and female patients who are equal to or older than 18 years of age and who have early diffuse systemic sclerosis (Disease duration < 18 months from the first non-Raynaud's symptom)

Patients with a modified Rodnans Skin Score (MRSS) of at least 20 in the absence of trunk involvement or a MRSS of at least 16 in patients with trunk involvement

*Exclusion Criteria:*

Concurrent connective tissue diseases other than systemic sclerosis

Significant pre-existing heart, liver, lungs, digestive system, blood and other diseases, cancer

Concurrent medical therapies (or during last 6 weeks before first dosing) that may potentially influence outcome of the study

Allergic to the study medication

**Example 8: Testing in CD45RB high-transfer mouse model (TNBS-induced Colitis) of Crohn's disease**

Male SJL mice are challenged via rectal catheter with TNBS to induce inflammation in the colon characterized by intense hyperemia, edema, and gut wall thickening. Dosing with Compound A begins on day 0 and continues until day 4. Mice are weighed on study days 0-4. On study day 4, mice are necropsied. Colons are removed, measured for length, and assessed for gross morphologic changes. Hemorrhage, stricture formation, ulceration, fecal blood, mucus, and diarrhea are scored at necropsy according to the following criteria:

0 = normal, no blood observed
1 = minimal/mild damage, hint of blood, soft stool
2 = moderate/marked damage, blood tinged, soft to liquid stool
3 = severe damage, bloody content, liquid stool or no stool

**Example 9: A 8-week, randomized, double-blind, placebo-controlled phase 2 study to evaluate the efficacy and safety of Compound A in subjects with moderate to severe Crohn's Disease who have failed or are intolerant to anti-tumor necrosis factor-alpha therapy**

| | |
|---|---|
| Study design: | Allocation: Randomized |
| Endpoint Classification: | Safety/Efficacy Study |
| Intervention Model: | Parallel Assignment |
| Masking: | Double Blind (Subject, Caregiver, Investigator, Outcomes Assessor) |
| Primary Purpose: | Treatment |

*Primary Outcome Measures:*

The primary endpoint of the study is Crohn's Disease Activity Index (CDAI) response at Week 8, defined by either a CDAI score of < 150 or a CDAI reduction from baseline of at least 100 points [ Time Frame: Week 8 ] [ Designated as safety issue: No ]

*Secondary Outcome Measures:*

Crohn's Disease Activity Index (CDAI) remission at Week 8, as defined by a CDAI score of < 150 [ Time Frame: Week 8 ] [ Designated as safety issue: No ]
A reduction of at least 100 points from baseline in Crohn's Disease Activity Index (CDAI) at Week 8 [ Time Frame: Week 8 ] [ Designated as safety issue: No ]
A reduction of at least 70 points from baseline in Crohn's Disease Activity Index (CDAI) at Week 8 [ Time Frame: Week 8 ] [ Designated as safety issue: No ]
Crohn's Disease Activity Index (CDAI) response (either remission defined by CDAI < 150 or a CDAI reduction from baseline of at least 100 points from baseline) at Week 12 [ Time Frame: Week 12 ] [ Designated as safety issue: No ]
Change from baseline Crohn's Disease Activity Index (CDAI) at Week 8

[ Time Frame: Week 8 ] [ Designated as safety issue: No ]

Pharmacokinetic (PK) evaluation of multiple doses of compound A [ Time Frame: Week 0 (Visit 3), Week 4 (Visit 5), Week 8 (Visit 6), Week 24 (Visit 10), Week 112 (Visit 32) predose; Week 0 (Visit 3), Week 4 (Visit 5), postdose ] [ Designated as safety issue: No ]
Immunogenicity (IM) evaluation of multiple doses of compound A [ Time Frame: Week 0 (Visit 3), Week 8 (Visit 6), Week 24 (Visit 10), Week 112(Visit 32) postdose ] [ Designated as safety issue: No ]

*Eligibility*

| | |
|---|---|
| Ages Eligible for Study: | 18 Years to 65 Years |
| Genders Eligible for Study: | Both |
| Accepts Healthy Volunteers: | No |

*Inclusion Criteria:*

Diagnosed ileal, ileo-colonic, or colonic CD at least 6 months prior to screening.
Men or women age 18 - 65 years at the time of screening.
Moderate-sever active Crohn's Disease (CD), defined by a Crohn's Disease Activity Index (CDAI) score higher or equal 220 and lower or equal 450 at Day 1.
No known history of active tuberculosis (TB).
Received at least one anti-TNFa agent for the treatment of CD and did not initially respond.

*Exclusion Criteria:*

Pregnant or breastfeeding women.
Presence of ileostomy and/or colostomy.
Short bowel syndrome.
Bowel perforation or obstruction.
History of cancer.

**Example 10: Testing in CD45RB high-transfer mouse model (Dextran Sulfate Induced Colitis) of Ulcerative Colitis**

Male SJL mice are exposed to 3% DSS in water for 5, 7 or 8 days to induce inflammation and gland loss with erosion in the colon. Dosing with Compound A begins on day 0 and continues until study termination. Mice are weighed and water consumption is measured daily. At necropsy, the entire colon from each mouse is removed and the length is determined. The colons are scored for gross changes using the following criteria:

0 = Normal
1 = Few formed pellets to semi-solid stool
2 = Semi-solid to fluid stool with or without definite evidence of blood
3 = Bloody stool
4 = Bloody fluid
5 = No content

Animals that survive to study termination are included in the analysis of terminal colon lengths and scores.

**Example 11: A randomized, double blind, placebo controlled study to evaluate the efficacy, safety and tolerability of Compound A in patients with active, moderate to severe ulcerative colitis**

| | |
|---|---|
| Study design: | Allocation: Randomized |
| Endpoint Classification: | Safety/Efficacy Study |
| Intervention Model: | Parallel Assignment |
| Masking: | Double Blind (Subject, Investigator) |
| Primary Purpose: | Treatment |

*Primary Outcome Measures:*

Rate of induction of remission after 28 days of treatment (using the Partial Mayo Score and the Modified Baron Score), also an Endoscopic biopsy will be taken. [ Time Frame: Partial Mayo Score throughout entire study, biopsy at end of dosing period ] [ Designated as safety issue: No ]

*Secondary Outcome Measures:*

Safety and tolerability assessments (vital signs, electrocardiogram [ECG], blood samples, serious adverse events, adverse events) [ Time Frame: Throughout entire study ] [ Designated as safety issue: Yes ]
Measurement of drug concentrations in blood [ Time Frame: During the dosing period only ] [ Designated as safety issue: No ]
Relationship between drug concentration in blood and disease activity [ Time Frame: Dosing period only ] [ Designated as safety issue: No ]

*Eligibility*

| | |
|---|---|
| Ages Eligible for Study: | 18 Years to 75 Years |
| Genders Eligible for Study: | Both |

(continued)

| | |
|---|---|
| Accepts Healthy Volunteers: | No |

*Inclusion Criteria:*

18-75 years males and females
Active, moderate to severe disease
Poor response to or no toleration of conventional therapy (e.g. steroids, mesalamine)

*Exclusion Criteria:*

Allergy to the drug
Very low or high body weight
Ongoing treatment with specific other medication (e.g. antibiotics)
Diagnosis of primary sclerosing cholangitis
Renal impairment
Toxic megacolon
Presence or history of specific other diseases, cancer, cardiac abnormalities, abnormal laboratory findings
History of alcohol or drug abuse
Positive HIV, Hepatitis B or Hepatitis C test result

**Example 12: Experimental autoimmune encephalomyelitis (EAE) mouse model of Multiple Sclerosis**

Male SJL mice with experimental autoimmune encephalomyelitis (EAE) are used in the study. The animals are randomized by body weight into one of the treatment groups or an untreated control group. Treatments with Compound A are initiated after randomization and continue for 12 weeks. Animals are weighted daily and scored on a standardized 5-point EAE disability scale. At the end of 12 weeks, mice are necropsied. Tissues are removed, post-fixed overnight in 4% PFA, dehydrated overnight in 30% sucrose, and then embedded for sectioning and immunostaining.

**Example 13: A 6-month, double-blind, randomized, placebo-controlled, multicenter study evaluating efficacy and safety of Compound A in patients with relapsing multiple sclerosis**

| | |
|---|---|
| Study design: | Allocation: Randomized |
| Endpoint Classification: | Safety/Efficacy Study |
| Intervention Model: | Parallel Assignment |
| Masking: | Double Blind (Subject, Investigator, Outcomes Assessor) |
| Primary Purpose: | Treatment |

*Primary Outcome Measures:*

Number of Patients Free of Gadolinium-enhanced T 1 -Weighted Magnetic Resonance Imaging (MRI) Lesions at Both Month 3 and Month 6 [ Time Frame: Month 3 and Month 6 ] [ Designated as safety issue: No ]

*Secondary Outcome Measures:*

Number of Patients Free of MS Relapse up to Month 6 (Confirmed Relapse Only) [ Time Frame: up to Month 6 ] [ Designated as safety issue: No ]
Number of Patients Free of New or Newly Enlarged T2 Lesions [ Time Frame: up to Month 3 and up to Month 6 ] [ Designated as safety issue: No ]
Annualized Relapse Rate (ARR) at 6 Months [ Time Frame: 6 Months ] [ Designated as safety issue: No ]

*Eligibility*

| | |
|---|---|
| Ages Eligible for Study: | 18 Years to 60 Years |
| Genders Eligible for Study: | Both |
| Accepts Healthy Volunteers: | No |

*Inclusion Criteria:*

Male and female patients aged 18-60

Patients with a diagnosis of multiple sclerosis

*Exclusion Criteria:*

Patients with a history or presence of chronic disease of the immune system other than MS

Patients with a history or presence of malignancy, pulmonary or heart disease, etc.

Pregnant or nursing (lactating) women

**Example 14: NMO/experimental autoimmune encephalomyelitis (EAE) mouse model of Neuromyelitis optica**

Male SJL mice with experimental autoimmune encephalomyelitis (EAE) are used in the study. The animals are randomized by body weight into one of the treatment groups or an untreated control group. Treatments with Compound A are initiated after randomization and continue for 12 weeks. Animals are weighted daily and scored on a standardized 5-point EAE disability scale. At the end of 12 weeks, mice are necropsied. AQP4-expressing tissues are removed, post-fixed overnight in 4% PFA and dehydrated overnight in 30% sucrose. Tissues are then embedded for sectioning and immunostaining.

**Example 15: An open label study of the effects of 12 month treatment of Compound A in neuromyelitis optica**

| | |
|---|---|
| Study design: | Endpoint Classification: Safety/Efficacy Study |
| Intervention Model: | Single Group Assignment |
| Masking: | Open Label |
| Primary Purpose: | Treatment |

*Primary Outcome Measures:*

Median Number of Neuromyelitis Optica (NMO) Attacks Per Year [ Time Frame: baseline, after 12 months of treatment ] [ Designated as safety issue: No ]

*Secondary Outcome Measures:*

Number Subjects Experiencing an NMO Attack in 12 Months of compound A Treatment [ Time Frame: 12 months ] [ Designated as safety issue: No ]

Change in Expanded Disability Status Scale (EDDS) Score [ Time Frame: baseline, 12 months ] [ Designated as safety issue: No ] The EDSS is an ordinal clinical rating scale ranging from 0 (normal neurologic examination) to 10 (death) in half-point increments.

Number of Subjects With Change in Visual Acuity in at Least One Eye by at Least One Point [ Time Frame: 12 months ] [ Designated as safety issue: No ] Visual acuity is measured using the the Visual Acuity subscale of the Opticospinal Impairment Score (OSIS) for Exacerbations. This subscale ranges from 0 (normal) to 8 (no light perception).

Number of Subjects With Change in Ambulation by at Least 1 Point [Time Frame: 12 months] [Designated as safety issue: No] Ambulation is measured by the Hauser Ambulation Index, which ranges from 0 (asymptomatic; fully active) to 9 (restricted to wheelchair; unable to transfer self independently.)

Mean Serum Concentration of compound A [Time Frame: 6 weeks, 3 months, 6 months, 9 months, 12 months] [Designated as safety issue: No]

Percentage Hemolysis [Time Frame: baseline, 6 weeks, 3 months, 6 months, 9 months, 12 months] [Designated as safety issue: No] Percentage of hemolysis is a measure of complement activity. Less than 20% lysis is deemed to

be complete complement inhibition.

Compound A mean concentration in Cerebrospinal Fluid (CSF) [Time Frame: 3 months] [Designated as safety issue: No]

Mean Complement Protein 5 (C5) Concentration in CSF [Time Frame: baseline, 3 months] [Designated as safety issue: No]

*Eligibility*

| | |
|---|---|
| Ages Eligible for Study: | 18 Years and older |
| Genders Eligible for Study: | Both |
| Accepts Healthy Volunteers: | No |

*Inclusion Criteria:*

Diagnosis of NMO, as defined by 2006 criteria OR NMO seropositive spectrum disorder (Recurrent ON or longitudinally extensive transverse myelitis (LETM)). All patients are NMO-IgG seropositive.

Clinical evidence of at least 2 relapses in last 6 months or 3 relapses in the last 12 months (with at least 1 relapse occurring in the preceding 6 months).

Age ≥18 years

*Exclusion Criteria:*

Candidates will be excluded from study entry if any of the following criteria are met at the time of randomization: Progressive neurological deterioration unrelated to relapses of ON or myelitis.

Pregnant, breastfeeding, or intending to conceive during the course of the study.

Patients will not participate in any other clinical therapeutic study or will not have participated in any other experimental treatment study within 30 days of screening.

Patients with a history of splenectomy, because of a potential increased risk of developing meningococcal infection.

**Example 16: Experimental autoimmune uveitis rat model of Optic Neuritis**

The optic nerve is a frequent site of involvement in multiple sclerosis (MS), and optic neuritis often appears as the presenting sign of MS. Many attempts have been made to create animal models of demyelinating optic neuritis, such as that found in MS, using autoimmunity or virus infection, and a review of these animal models has been presented by Levkovitch-Verbin (Animal models of optic nerve diseases. Eye (2004) 18, 1066-1074. doi:10.1038/sj.eye.6701576). Lewis rat model of experimental autoimmune uveitis is used in the study. The animals are randomized by body weight into one of the treatment groups or an untreated control group. Compound A is prepared as an oral suspension and dosed orally, once per day at two doses, 10 and 20 mg/kg. The efficacy is compared to animals that received the positive control cyclosporine dosed at 25 mg/kg. This dose of cyclosporine is designed to inhibit the model by 95 -100% and is equivalent to an exposure that is not tolerated in humans. Efficacy in the model is assessed by clinical grading and by assessment of the sections of the eyes prepared for histology at the completion of the experiment.

**Example 17: A double blind randomized control trial of Compound A in optic neuritis**

| | |
|---|---|
| Study design: | Allocation: Randomized |
| Endpoint Classification: | Safety/Efficacy Study |
| Intervention Model: | Parallel Assignment |
| Masking: | Double Blind (Subject, Caregiver, Investigator, Outcomes Assessor) |
| Primary Purpose: | Treatment |

*Primary Outcome Measures:*

Scanning laser polarimetry determined retinal nerve fibre layer thickness [Time Frame: Baseline, 6 and 12 months] [Designated as safety issue: No]

The primary outcome will be difference in retinal nerve fibre thickness at 6 months between affected eye and non-affected fellow eye at baseline between the amiloride and placebo group.

An additional measure will be made at 12 months.

*Secondary Outcome Measures:*

Optical coherence tomography determined difference in retinal nerve fibre layer thickness. [Time Frame: Baseline, 6 and 12 months] [Designated as safety issue: No] Difference in thickness at 6 months and 12 months between affected eye and non-affected fellow eye at baseline, between the test and placebo group

Differences between the test and placebo groups in non-conventional MRI surrogate marker of white matter and grey matter injury and connectivity by 3T scanning. [Time Frame: Baseline, 6 and 12 months] [Designated as safety issue: No]

a. Diffusion weighted imaging (DWI) - measures; fractional anisotropy (FA), mean diffusivity (MD), axial and radial diffusivity (RD) of the posterior visual tracts
b. High Resolution T1-weighted image measure of grey matter volume
c. Magnetic resonance spectroscopy (MRS) measures of N-acetyl aspartate (NAA) in the visual cortex
d. Resting state functional MRI (RS fMRI) patterns of activity
e. Magnetisation transfer imaging (MTI) derived magnetisation transfer ratio (MTR) of the white and grey matter

Visual Function [Time Frame: Baseline, 6 and 12 months]
[Designated as safety issue: No]

a. High and low contrast (2.5% and 1.25%) visual acuity.
b. Farnsworth Munsell 100 Hue (FM100) colour vision test.

Visual Electrophysiology [Time Frame: 0 and 6 months] [Designated as safety issue: No]
Differences in visually evoked potential and pattern electro-retinogram between the amiloride and placebo groups as additional measures of visual function

Quality of life questionnaires [Time Frame: Baseline, 6 and 12 months] [Designated as safety issue: No]

a. 25 point national institutes for health visual function questionnaire
b. 10 point neuro ophthalmic supplement

*Eligibility*

| | |
|---|---|
| Ages Eligible for Study: | 18 Years to 55 Years |
| Genders Eligible for Study: | Both |
| Accepts Healthy Volunteers: | No |

*Inclusion Criteria:*

Participants with a first episode of unilateral optic neuritis

Participants with an existing diagnosis of relapsing remitting MS and new onset of ON are eligible if they have;

a. Not had a previous episode of optic neuritis,
b. A duration of disease of ≤10 years
c. An EDSS (Expanded Disability Status Scale) of ≤3.
d. No immune modulating treatment other than β-Interferon or glatiramer acetate at time of recruitment

Able to be randomised within 28 days of onset of visual symptoms

Visual acuity of ≤6/9

Participant is willing and able to give informed consent for participation in the study and able to comply with study visits

Male or Female, aged between18 - 55 years.

Stable dose of current regular medication for at least 4 weeks prior to study entry.

Participant has clinically acceptable urea and electrolytes and estimated glomerular filtration rate (eGFR) >60

*Exclusion Criteria:*

Previous diagnosis of optic neuritis

Any concomitant immune suppressing or immune modulating therapy excluding β-interferon or glatiramer acetate.

Female participants who are pregnant, lactating or planning pregnancy during the course of the study.

Any contra-indication to MRI - severe claustrophobia, metal implant, pacemaker, etc.

Participant who is terminally ill or is inappropriate for placebo medication

Impaired renal function : eGFR ≤60, anuria, acute or chronic renal insufficiency and evidence of diabetic nephropathy

Raised serum potassium ($K^+$ >5.5mmol/l)

Diabetes

Significant concomitant eye disease in either eye that may affect diseased or fellow eye results.

Any other significant disease or disorder which, in the opinion of the investigator, may either put the participants at risk because of participation in the study, or may influence the result of the study, or the participant's ability to participate in the study.

Participants who have participated in another research study involving an investigational product in the past 12 weeks.

**REFERENCE Example 18: Testing in MRL/lpr mouse model of lupus**

Compound A was tested for efficacy in the MRL/lpr mouse model of lupus (Shlomchik MJ, et al, 1994, J Exp Med 180:1295-1306; Cohen PL and Eisenberg R. A. 1991, Annu Rev Immunol, 9:243-69; Honigberg, L.A., et al., 2010, Proc Natl Acad Sci U S A. 107:13075-80). Female MRL/MpJ-Tnfrsf6lpr/J mice of 8-9 weeks of age were used in the study. When the mice reached 12 weeks of age, they were randomized by animal body weight into one of the treatment groups or an untreated control group. Treatments was initiated after randomization and continued for 12 weeks. Starting on study week 1, and then every week thereafter, urine from each animal was tested for proteinuria using the Clinitech Multistick test strip (Bayer). The animals were observed daily for significant clinical signs, moribundity and mortality. Scoring of lymphadenopathy (cervical, brachial, and inguinal) for all animals was recorded every week once lesions become apparent in 50% of the animals in vehicle-treated group.

Compound A was prepared as an oral suspension. Initial dosing was completed at two dose levels, 60 and 90 mg/kg, BID for a total dose of 120 and 180 mg/kg/day. Separate, independent pharmacokinetics experiments were done and showed an unexpectedly low and insufficient exposure of compound A in mice at the 60 mg/kg dose. Therefore the lower dose was doubled after the initial 4 weeks of dosing. Dosing for the final 8 weeks was at 180 and 240 mg/kg/day.

Compound A demonstrated good efficacy in the model in multiple rounds of efficacy measurements. Both doses demonstrating a significant reduction in protein in the urine (Fig. 7), a reduction in lymphadenopathy score (Fig. 8), in mean absolute spleen weight (Fig. 9) and in mean lymph node weight (Fig. 10).

Clinical chemistry values from the animals showed a dose-dependent reduction in blood urea nitrogen (BUN) values, suggesting that kidney damage was lessened by treatment. The data in Table 3 illustrates that the reduction in BUN is statistically significant at the higher dose. In the two treatments groups, 21/23 (91%) of the animals were found to have a BUN ≤ 33 mg/dL (in the normal range of BUN values for the mouse) versus only 2/11 (18%) in the control, untreated group.

**Table 3**

| **Key Clinical Chemistry Values** | | | | | |
|---|---|---|---|---|---|
| Treatment (p.o. BID) | BUN (mg/dL) | BUN p value | Creatinine | BUN/ Creatinine | BUN/ Creatinine p value |
| Vehicle | 71.7 | - | 0.24 | 270.9 | - |

(continued)

| Key Clinical Chemistry Values | | | | | |
|---|---|---|---|---|---|
| Treatment (p.o. BID) | BUN (mg/dL) | BUN p value | Creatinine | BUN/ Creatinine | BUN/ Creatinine p value |
| Compound A, 90 mg/kg | 52.4 | 0.5 | 0.26 | 163.9 | .05 |
| Compound A,120 mg/kg | 29.5 | 0.04 | 0.20 | 147.7 | .03 |

In conclusion, compound A in the MRL/lpr mouse model of SLE demonstrated a significant reduction in multiple measures of efficacy including protein in the urine, lymphadenopathy score, spleen weight and lymph node weight.

**REFERENCE Example 19: Testing in Experimental Autoimmune Encephalitis**

Compound A was tested in the experimental autoimmune encephalitis (EAE) model in Lewis rats. EAE was induced by MBP69-88/CFA immunization and pertussis toxin injection in Lewis rats (Hashim, et al., 1986, J Neurosci Res.;16(3):467-78). Compound A was prepared as an oral suspension and dosed orally, twice per day (BID) at three doses, 7.5 and 15 and 30 mg/kg for total daily doses of 15, 30 and 60 mg/kg. The efficacy was compared to animals that received the positive control FTY720 (also known as fingolimod; a compound approved for use in humans) dosed once per day at a dose of 0.5 mg/kg. The treatment started on Day 8, when 48% of the rats had signs of EAE. Compound A showed excellent efficacy and a clear dose response. At 15 and 30 mg/kg BID, it significantly reduced maximum EAE severity as well as end severity compared to the vehicle control group, see Figure 11. At the highest dose (30 mg/kg BID) it significantly reduced EAE incidence (Table 4). These results demonstrate that Compound A was efficacious at reducing EAE severity in the study in a dose-dependent manner.

**Table 4**

| Group | Treatment (p.o., all groups) | EAE incidence (%) | Median day of onset (all rats) | Mean day of onset +/- SEM (sick rats) |
|---|---|---|---|---|
| 1 | Vehicle, BID | 100.0% | 9.0 | 9.3 +/- 0.6 |
| 2 | FTY720, 0.5 mg/kg, QD | 87.5% | 8.0 | 8.0 +/- 0.3 |
| 3 | Compound A, 7.5 mg/kg, BID | 100.0% | 9.0 | 9.0 +/- 0.5 |
| 4 | Compound A, 15 mg/kg, BID | 100.0% | 8.0 | 8.5 +/- 0.4 |
| 5 | Compound A, 30 mg/kg, BID | 50.0%* | 13.5 | 9.0 +/- 0.9 |
| *p<0.05 vs. vehicle | | | | |

In conclusion, compound A in the experimental autoimmune encephalitis model demonstrated a dose-dependent decrease in clinical score and at the highest dose employed, appeared superior to fingolimod, a compound approved for use in humans.

**REFERENCE Example 20: Testing in model of Uveitis**

The animal model used in this testing was the Lewis rat model of experimental autoimmune uveitis, a well-known model of uveitis (Nussenblatt RB, et al, 1981, J Clin Invest. 67(4): 1228-1231; Mochizuki M, et al, 1985, Invest Ophthalmol Vis Sci. 26(2): 226-232). Compound A was prepared as an oral suspension and dosed orally, once per day at two doses, 10 and 20 mg/kg. The efficacy was compared to animals that received the positive control cyclosporine dosed at 25 mg/kg. This dose of cyclosporine was designed to inhibit the model by 95 -100% and is equivalent to an exposure that is not tolerated in humans. Efficacy in the model was assessed by clinical grading and by assessment of the sections of the eyes prepared for histology at the completion of the experiment. Compound A was effective in this model showing a dose dependent decrease in disease severity as assessed by both clinical score (Fig. 12), and histopathological score (Fig. 13).

In conclusion, compound A in an experimental autoimmune model of uveitis elicited a greater than 50% reduction

in clinical scores and a greater than 75% reduction in histopathological scores at very modest doses.

**REFERENCE Example 21: Evaluate the efficacy and safety of Compound A compared to placebo in subjects with chronic, moderately-to-severely active systemic lupus erythematosus (SLE)**

| | |
|---|---|
| Study design: | Allocation: randomized |
| Endpoint Classification: | Safety/Efficacy Study |
| Intervention Model: | Parallel Assignment |
| Masking: | Double Blind (Subject, Caregiver, Investigator, Outcomes Assessor) |
| Primary Purpose: | Treatment |

**Primary Outcome Measures:**

Achievement of response in a systemic lupus erythematosus (SLE) responder index [Time Frame: Day 169 (or 6 months)] [Designated as safety issue: No] Number and percentage of participants achieving a response in an SLE responder index at Day 169 (or 6 months)

**Secondary Outcome Measures:**

Achievement of response in a systemic lupus erythematosus (SLE) responder index [Time Frame: Day 365 (or 1 year)] [Designated as safety issue: No]
Number and percentage of participants achieving a response in an SLE responder index at Day 365 (or 1 year).

| Arms | Assigned Interventions |
|---|---|
| Experimental: Compound A Low-dose Group Compound A will be given at the predetermined dosing intervals as specified in the protocol | Low dose of Compound A administered at the predetermined intervals |
| Experimental: Compound A High-dose Group Compound A will be given at the predetermined dosing intervals as specified in the protocol | High dose of Compound A administered at predetermined intervals |
| Placebo Comparator: Matching Placebo Group Placebo matching Compound A will be given at the predetermined dosing intervals as specified in the protocol | Other: Placebo Matching placebo to Compound A administered at predetermined intervals |

Eligibility

| | |
|---|---|
| Ages Eligible for Study: | 18 Years to 75 Years |
| Gender Eligible for Study: | Both |
| Accepts Healthy Volunteers: | No |

**Inclusion Criteria:**

- Fulfills at least 4 of the 11 American College of Rheumatology (ACR) criteria for systemic lupus erythematosus (SLE) including a positive antinuclear antibody (ANA) greater than or equal to 1:80 or elevated anti-double-stranded DNA or anti-Smith antibody at screening
- Pediatric or adult SLE with chronic disease activity for greater than or equal to 24 weeks
- Weight greater than or equal to 40 kg
- Active moderate to severe SLE disease based on SLE disease activity score (SLEDAI) and British Isles Lupus

Assessment Group Index (BILAG) and Physicians Global Assessment

- No evidence of cervical malignancy on Pap smear within 2 years of randomization
- Female subjects must be willing to avoid pregnancy
- Negative tuberculosis (TB) test or newly positive TB test due to latent TB for which treatment must be initiated at or before randomization

**Exclusion Criteria:**

- Active severe SLE-driven renal disease or unstable renal disease prior to screening
- Active severe or unstable neuropsychiatric SLE
- Clinically significant active infection including ongoing and chronic infections
- History of human immunodeficiency virus (HIV)
- Confirmed Positive tests for hepatitis B or positive test for hepatitis C
- History of severe herpes infection such as herpes encephalitis, ophthalmic herpes, disseminated herpes
- Live or attenuated vaccine within 4 weeks prior to screening
- Subjects with significant hematologic abnormalities

**REFERENCE Example 22: Evaluate the efficacy and safety of Compound A in subjects with Uveitis**

| | |
|---|---|
| Study design: | Allocation: randomized |
| Endpoint Classification: | Safety/Efficacy Study |
| Intervention Model: | Parallel Assignment |
| Masking: | Double Blind (Subject, Caregiver, Investigator, Outcomes Assessor) |
| Primary Purpose: | Treatment |

**Primary Outcome Measures:**

- Control of intraocular inflammation [Time Frame: at 6-month visit] [Designated as safety issue: No]
  Absence of intraocular inflammation (e.g. less than trace AC cells; no vitreous haze; inactive chorioretinal lesions).
- Evaluation of Adverse Events [Time Frame: Baseline to Final Visit (Final Visit could occur at any point up to 282 weeks)] [Designated as safety issue: Yes]
- Significant laboratory value changes [Time Frame: Baseline to Final Visit (Final Visit could occur at any point up to 282 weeks)] [Designated as safety issue: Yes]
- Significant vital sign changes [Time Frame: Baseline to Final Visit (Final Visit could occur at any point up to 282 weeks)] [Designated as safety issue: Yes]

**Secondary Outcome Measures:**

- Control of intraocular inflammation [Time Frame: 12-month clinical visit] [Designated as safety issue: No]
- Proportion of subjects at each study time point with no new active, inflammatory chorioretinal or inflammatory retinal vascular lesion in both eyes relative to Baseline for subjects who had inactive uveitis when they entered the study. [Time Frame: Final Visit (Final Visit could occur at any point up to 282 weeks)] [Designated as safety issue: No]
- Proportion of subjects at each study time point with no new active, inflammatory chorioretinal or inflammatory retinal vascular lesion in both eyes relative to Week 8 for subjects who had active uveitis when they entered the study. [Time Frame: Final Visit (Final Visit could occur at any point up to 282 weeks)] [Designated as safety issue: No]
- Proportion of subjects at each study time point with a Grade <= 0.5+ in AC cells in both eyes on Slit Lamp Exam according to SUN criteria. [Time Frame: Final Visit (Final Visit could occur at any point up to 282 weeks)] [Designated as safety issue: No]
- Proportion of subjects at each study time point with a Grade <= 0.5+ in vitreous haze in both eyes on indirect

ophthalmoscopy according to NEI/SUN criteria. [Time Frame: Final Visit (Final Visit could occur at any point up to 282 weeks)] [Designated as safety issue: No]

- Proportion of subjects at each study time point without a worsening of BCVA by >= 15 letters on the ETDRS in both eyes relative to Baseline for subjects who had inactive uveitis when they entered the study. [Time Frame: Final Visit (Final Visit could occur at any point up to 282 weeks)] [Designated as safety issue: No]
- Proportion of subjects at each study time point without a worsening of BCVA by >= 15 letters on the ETDRS in both eyes relative to Week 8 for subjects who had active uveitis when they entered the study. [Time Frame: Final Visit (Final Visit could occur at any point up to 282 weeks)] [Designated as safety issue: No]
- Percent change in central retinal thickness (1 mm subfield) in each eye at each study time point relative to Baseline for subjects who had inactive uveitis when they entered the study. [Time Frame: Baseline to Final Visit (Final Visit could occur at any point up to 282 weeks)] [Designated as safety issue: No]
- Percent change in central retinal thickness (1 mm subfield) in each eye at each study time point relative to Week 8 for subjects who had active uveitis when they entered the study. [Time Frame: Week 8 to Final Visit (Final Visit could occur at any point up to 282 weeks)] [Designated as safety issue: No]
- Change in NEI Visual Functioning Questionnaire (VFQ-25) score at each study time point relative to Baseline for subjects who had inactive uveitis when they entered the study. [Time Frame: Baseline to Final Visit (Final Visit could occur at any point up to 282 weeks)] [Designated as safety issue: No]
- Change in NEI Visual Functioning Questionnaire (VFQ-25) score at each study time point relative to week 8 for subjects who had active uveitis when they entered the study. [Time Frame: Week 8 to Final Visit (Final Visit could occur at any point up to 282 weeks)] [Designated as safety issue: No]
- Proportion of subjects at each study time point achieving a >= 50% reduction in immunosuppression load relative to Baseline for subjects who had inactive uveitis when they entered the study. [Time Frame: Final Visit (Final Visit could occur at any point up to 282 weeks)] [Designated as safety issue: No]
- Proportion of subjects at each study time point achieving a >= 50% reduction in immunosuppression load relative to Week 8 for subjects who had active uveitis when they entered the study. [Time Frame: Final Visit (Final Visit could occur at any point up to 282 weeks)] [Designated as safety issue: No]

**Other Outcome Measures:**

- Elevation of IOP [Time Frame: At 3-month, 6-month, and 12-month visit] [Designated as safety issue: Yes] Ocular hypertension and IOP>30 and 10 mm Hg increase or greater in IOP will be assessed.
- Progression of cataract or need for cataract surgery [Time Frame: At 3-month, 6 month, and 12-month visit] [Designated as safety issue: Yes]

| Arms | Assigned Interventions |
|---|---|
| Experimental: Compound A Low-dose Group Compound A will be given at the predetermined dosing intervals as specified in the protocol | Low dose of Compound A administered at the predetermined intervals |
| Experimental: Compound A High-dose Group Compound A will be given at the predetermined dosing intervals as specified in the protocol | High dose of Compound A administered at predetermined intervals |
| Placebo Comparator: Matching Placebo Group Placebo matching Compound A will be given at the predetermined dosing intervals as specified in the protocol | Other: Placebo Matching placebo to Compound A administered at predetermined intervals |

**Detailed Description:**

Background: Intermediate and posterior uveitis are thought to be severe intraocular inflammation that may lead to permanent visual loss. It is estimated that these forms of uveitis comprise the fifth or sixth leading cause of blindness and tend to affect working class age patients, thus causing loss of work hours and diminished productivity and quality of life. Because the posterior segment of the eye is not adequately treated by corticosteroid drops often systemic drug therapy is used including oral corticosteroids or prednisone. Prednisone can have a myriad of side effects in approximately

one-quarter to one-third of cases treated in tertiary care centers such as ours, additional medications such as immunosuppressive drugs are required to control the disease and/or to allow for appropriate tapering of oral prednisone to subsequent levels that have a low side effect profile when delivered over a long period of time. Typically, chronic prednisone therapy in doses of 7.5 mg daily or less are thought to have a low enough side effect profile to be amenable to long-term therapy. However frequently immunosuppressive drugs are required to get the dosing to this level. There are occasions when patients are intolerant of any dose of oral corticosteroids or are intolerant of the higher doses of oral corticosteroids (30 - 60 mg daily) and therefore this treatment modality is avoided due to prednisone's attendant side effects. Although periocular and intravitreal corticosteroids injections may be performed, with these modalities the standard of care is to wait until the disease reactivates before instituting such therapy and therefore a chronic suppressive dose is not obtained. The fluocinolone acetonide implant (Retisert®, Bausch and Lomb, Tampa, FL) is FDA-approved for the treatment of intermediate and posterior uveitis and it is equally effective in controlling uveitis as high-dose oral corticosteroids but avoids the systemic side effects associated with the use of high doses of oral corticosteroids. However, this form of local therapy has high rates of ocular side effects, including ocular hypertension causing glaucoma and/or requiring glaucoma surgery and cataracts. Furthermore, every two and half to three years the implant is exhausted of corticosteroid and therefore repeat surgical insertion of another implant may be required. Therefore, an orrally administered, non-steroid based therapy that effectively treats uvetis is highly desirable.

**Eligibility**

| | |
|---|---|
| Ages Eligible for Study: | 18 Years and older |
| Gender Eligible for Study: | Both |
| Accepts Healthy Volunteers: | No |

**Inclusion Criteria:**

- Active sight-threatening intermediate or posterior uveitis.
- Patients must be age 18 years or older and sign an informed consent.
- The ocular media must be clear enough to obtain OCT and fundus photographs.
- No elective intraocular surgery should be planned for the first 3 months after enrollment.

**Exclusion Criteria:**

- Infectious uveitis
- History of scleritis
- Active or suspected viral infection of the cornea or conjunctiva
- History of mycobacterial or fungal disease
- HIV positivity
- Age <18 years old
- Uncontrolled IOP
- Advanced glaucoma
- Aphakia with rupture of the posterior lens capsule
- ACIOL with rupture of the posterior lens capsule
- Media opacity that would preclude evaluation of the posterior pole via fundus photography or OCT assessment
- Planned elective ocular surgery within 3 months of enrollment

**REFERENCE Example 23: Evaluate the efficacy and safety of Compound A in subjects with Autoimmune Encephalitis**

Study design: Allocation: randomized

(continued)

| | |
|---|---|
| Endpoint Classification: | Safety/Efficacy Study |
| Intervention Model: | Parallel Assignment |
| Masking: | Double Blind (Subject, Caregiver, Investigator, Outcomes Assessor) |
| Primary Purpose: | Treatment |

**Primary Outcome Measures:**

- To assess the safety and tolerability of Compound A in the treatment of Autoimmune Encephalitis. [Time Frame: 12 months] [Designated as safety issue: Yes]
- The natural history of Autoimmune Encephalitis is a progressive deterioration in cortical function; therefore, any evidence of stabilization or improvement in measures of motor function, cognition and/or seizure frequency will be evidence of efficacy and will be assessed at [Time Frame: 12 months] [Designated as safety issue: No]

**Secondary Outcome Measures:**

- The percentage of patients with a 50% reduction in seizure frequency (responder rate) at 6 months post treatment (as compared to the patient's baseline seizure frequency) will be determined. [Time Frame: 12 months] [Designated as safety issue: No]

| Arms | Assigned Interventions |
|---|---|
| Experimental: Compound A Low-dose Group Compound A will be given at the predetermined dosing intervals as specified in the protocol | Low dose of Compound A administered at the predetermined intervals |
| Experimental: Compound A High-dose Group Compound A will be given at the predetermined dosing intervals as specified in the protocol | High dose of Compound A administered at predetermined intervals |
| Placebo Comparator: Matching Placebo Group Placebo matching Compound A will be given at the predetermined dosing intervals as specified in the protocol | Other: Placebo Matching placebo to Compound A at predetermined intervals |

Eligibility

| | |
|---|---|
| Ages Eligible for Study: | 18 Years and older |
| Gender Eligible for Study: | Both |
| Accepts Healthy Volunteers: | No |

**Inclusion Criteria:**

- Encephalopathy symptoms (change of mental state and consciousness level) persist for more than 24 hours;
- At least one or more clinical features of the followings: fever, epilepsy, focal neurological deficiency symptoms, changes in CSF(cerebrospinal fluid inflammatory), changes in EEG (electroencephalogram), radiographic abnormalities;
- Clinical suspected encephalitis, but conventional detected methods cannot make etiology clear

**Exclusion Criteria:**

- The metabolic encephalopathy;
- Infectious encephalitis with clinically clear pathogen, referring the specific pathogenic microorganisms, including: bacteria, virus, fungus, parasite, spirochete and so on;
- Non-infectious encephalitis with clinically clear diagnosis, including: multiple sclerosis, optic neuromyelitis, acute disseminated encephalomyelitis and so on.

## Claims

**1.** A pharmaceutical composition comprising 5-{[(2S,5R)-2,5-Dimethyl-4-(tetrahydro-2H-pyran-4-ylmethyl)piperazin-1-yl]carbonyl}-N-(5-fluoro-2-methylpyrimidin-4-yl)-6,6-dimethyl-1,4,5,6-tetrahydropyrrolo[3,4-c]pyrazol-3-amine, or a pharmaceutically acceptable salt thereof,
for use in a method of treating an autoimmune or alloimmune disease in a patient, wherein the autoimmune or alloimmune disease is:

rheumatoid arthritis;
multiple sclerosis;
inflammatory bowel disease;
optic neuritis;
neuromyelitis optica;
Sjögren's syndrome;
systemic scleroderma;
ankylosing spondylitis;
autoimmune hepatitis;
graft vs host disease; or
organ transplant rejection.

**2.** The pharmaceutical composition for use according to claim 1, wherein the autoimmune or alloimmune disease is rheumatoid arthritis.

**3.** The pharmaceutical composition for use according to claim 2, wherein the rheumatoid arthritis is rheumatoid factor positive (seropositive) RA, rheumatoid factor negative (seronegative) RA, or juvenile RA.

**4.** The pharmaceutical composition for use according to claim 1, wherein the autoimmune or alloimmune disease is multiple sclerosis.

**5.** The pharmaceutical composition for use according to claim 4, wherein the multiple sclerosis is relapsing-remitting (RR) multiple sclerosis, secondary progressive (SP) multiple sclerosis, primary progressive (PP) multiple sclerosis, progressive relapsing multiple sclerosis, clinically isolated syndrome (CIS), or radiologically isolated syndrome (RIS).

**6.** The pharmaceutical composition for use according to claim 1, wherein the autoimmune or alloimmune disease is inflammatory bowel disease.

**7.** The pharmaceutical composition for use according to claim 6, wherein the inflammatory bowel disease is Crohn's disease, ulcerative colitis, collagenous colitis, lymphocytic colitis, diversion colitis, Behçet's disease, or indeterminate colitis.

**8.** The pharmaceutical composition for use according to claim 1, wherein the autoimmune or alloimmune disease is optic neuritis; wherein the optic neuritis is papillitis neuritis or retrobulbar neuritis.

**9.** The pharmaceutical composition for use according to claim 1, wherein the autoimmune or alloimmune disease is neuromyelitis optica.

**10.** The pharmaceutical composition for use according to claim 1, wherein the autoimmune or alloimmune disease is

Sjögren's syndrome.

11. The pharmaceutical composition for use according to claim 1, wherein the autoimmune or alloimmune disease is systemic scleroderma.

12. The pharmaceutical composition for use according to claim 1, wherein the autoimmune or alloimmune disease is ankylosing spondylitis.

13. The pharmaceutical composition for use according to claim 1, wherein the autoimmune or alloimmune disease is autoimmune hepatitis.

14. The pharmaceutical composition for use according to claim 1, wherein the autoimmune or alloimmune disease is graft vs host disease; wherein the graft vs host disease is acute graft vs host disease, or chronic graft vs host disease.

15. The pharmaceutical composition for use according to claim 1, wherein the autoimmune or alloimmune disease is organ transplant rejection.

**Patentansprüche**

1. Pharmazeutische Zusammensetzung, die 5-{[(2S,5R)-2,5-Dimethyl-4-(tetrahydro-2H-pyran-4-ylmethyl)piperazin-1-yl]carbonyl}-N-(5-fluor-2-methylpyrimidin-4-yl)-6,6-dimethyl-1,4,5,6-tetrahydropyrrolo[3,4-c]pyrazol-3-amin oder ein pharmazeutisch annehmbares Salz davon umfasst,
zur Verwendung in einem Verfahren zur Behandlung einer Autoimmun- oder Alloimmunerkrankung bei einem Patienten, wobei die Autoimmun- oder Alloimmunerkrankung:

    rheumatoide Arthritis;
    multiple Sklerose;
    chronisch-entzündliche Darmerkrankung;
    Optikusneuritis;
    Neuromyelitis optica;
    Sjögren-Syndrom;
    systemische Sklerodermie;
    ankylosierende Spondylitis;
    Autoimmunhepatitis;
    Transplantat-Wirt-Erkrankung; oder
    Organtransplantatabstoßung ist.

2. Pharmazeutische Zusammensetzung zur Verwendung nach Anspruch 1, wobei die Autoimmun- oder Alloimmunerkrankung rheumatoide Arthritis ist.

3. Pharmazeutische Zusammensetzung zur Verwendung nach Anspruch 2, wobei die rheumatoide Arthritis Rheumafaktor-positive (seropositive) RA, Rheumafaktor-negative (seronegative) RA oder juvenile RA ist.

4. Pharmazeutische Zusammensetzung zur Verwendung nach Anspruch 1, wobei die Autoimmun- oder Alloimmunerkrankung multiple Sklerose ist.

5. Pharmazeutische Zusammensetzung zur Verwendung nach Anspruch 4, wobei die multiple Sklerose schubförmig remittierende (RR) multiple Sklerose, sekundär progrediente (SP) multiple Sklerose, primär progrediente (PP) multiple Sklerose, progredient schubförmige multiple Sklerose, klinisch isoliertes Syndrom (CIS) oder radiologisch isoliertes Syndrom (RIS) ist.

6. Pharmazeutische Zusammensetzung zur Verwendung nach Anspruch 1, wobei die Autoimmun- oder Alloimmunerkrankung chronisch-entzündliche Darmerkrankung ist.

7. Pharmazeutische Zusammensetzung zur Verwendung nach Anspruch 6, wobei die chronisch-entzündliche Darmerkrankung Morbus Crohn, Colitis ulcerosa, kollagene Kolitis, lymphozytäre Kolitis, Diversionskolitis, Morbus Behçet oder unbestimmte Kolitis ist.

**8.** Pharmazeutische Zusammensetzung zur Verwendung nach Anspruch 1, wobei die Autoimmun- oder Alloimmunerkrankung Optikusneuritis ist; wobei die Optikusneuritis Papillitis-Neuritis oder Retrobulbärneuritis ist.

**9.** Pharmazeutische Zusammensetzung zur Verwendung nach Anspruch 1, wobei die Autoimmun- oder Alloimmunerkrankung Neuromyelitis optica ist.

**10.** Pharmazeutische Zusammensetzung zur Verwendung nach Anspruch 1, wobei die Autoimmun- oder Alloimmunerkrankung das Sjögren-Syndrom ist.

**11.** Pharmazeutische Zusammensetzung zur Verwendung nach Anspruch 1, wobei die Autoimmun- oder Alloimmunerkrankung systemische Sklerodermie ist.

**12.** Pharmazeutische Zusammensetzung zur Verwendung nach Anspruch 1, wobei die Autoimmun- oder Alloimmunerkrankung ankylosierende Spondylitis ist.

**13.** Pharmazeutische Zusammensetzung zur Verwendung nach Anspruch 1, wobei die Autoimmun- oder Alloimmunerkrankung Autoimmunhepatitis ist.

**14.** Pharmazeutische Zusammensetzung zur Verwendung nach Anspruch 1, wobei die Autoimmun- oder Alloimmunerkrankung eine Transplantat-Wirt-Erkrankung ist; wobei die Transplantat-Wirt-Erkrankung eine akute Transplantat-Wirt-Erkrankung oder eine chronische Transplantat-Wirt-Erkrankung ist.

**15.** Pharmazeutische Zusammensetzung zur Verwendung nach Anspruch 1, wobei die Autoimmun- oder Alloimmunerkrankung Organtransplantatabstoßung ist.

**Revendications**

**1.** Composition pharmaceutique comprenant une 5-{[(2S,5R)-2,5-diméthyl-4-(tétrahydro-2H-pyran-4-ylméthyl)pipérazin-l-yl]carbonyl}-N-(5-fluoro-2-méthylpyrimidin-4-yl)-6,6-diméthyl-1,4,5,6-tétrahydropyrrolo[3,4-c]pyrazol-3-amine, ou un sel pharmaceutiquement acceptable de celle-ci,
à utiliser dans un procédé de traitement d'une maladie auto-immune ou allo-immune chez un patient, dans laquelle la maladie auto-immune ou allo-immune est :

une arthrite rhumatoïde ;
une sclérose en plaques ;
une maladie intestinale inflammatoire ;
une névrite optique ;
une neuromyélite optique ;
un syndrome de Sjögren ;
une sclérodermie systémique ;
une spondylarthrite ankylosante ;
une hépatite auto-immune ;
une réaction du greffon contre l'hôte ; ou
un rejet de greffe d'organe.

**2.** Composition pharmaceutique à utiliser selon la revendication 1, dans laquelle la maladie auto-immune ou allo-immune est une polyarthrite rhumatoïde.

**3.** Composition pharmaceutique à utiliser selon la revendication 2, dans laquelle la polyarthrite rhumatoïde (RA) est une RA à facteur rhumatoïde positif (séropositive), une RA à facteur rhumatoïde négatif (séronégative), ou une RA juvénile.

**4.** Composition pharmaceutique à utiliser selon la revendication 1, dans laquelle la maladie auto-immune ou allo-immune est une sclérose en plaques.

**5.** Composition pharmaceutique à utiliser selon la revendication 4, dans laquelle la sclérose en plaques est une sclérose en plaques récurrente-rémittente (RR), une sclérose en plaques progressive secondaire (SP), une sclérose en

plaques progressive primaire (PP), une sclérose en plaques récurrente progressive, un syndrome cliniquement isolé (CIS), ou un syndrome radiologiquement isolé (RIS).

6. Composition pharmaceutique à utiliser selon la revendication 1, dans laquelle la maladie auto-immune ou allo-immune est une maladie intestinale inflammatoire.

7. Composition pharmaceutique à utiliser selon la revendication 6, dans laquelle la maladie intestinale inflammatoire est la maladie de Crohn, une colite ulcéreuse, une colite collagène, une colite lymphocytaire, une colite de diversion, la maladie de Behçet, ou une colite indéterminée.

8. Composition pharmaceutique à utiliser selon la revendication 1, dans laquelle la maladie auto-immune ou allo-immune est une névrite optique ; dans laquelle la névrite optique est une névrite papillite ou une névrite rétrobulbaire.

9. Composition pharmaceutique à utiliser selon la revendication 1, dans laquelle la maladie auto-immune ou allo-immune est une neuromyélite optique.

10. Composition pharmaceutique à utiliser selon la revendication 1, dans laquelle la maladie auto-immune ou allo-immune est le syndrome de Sjögren.

11. Composition pharmaceutique à utiliser selon la revendication 1, dans laquelle la maladie auto-immune ou allo-immune est une sclérodermie systémique.

12. Composition pharmaceutique à utiliser selon la revendication 1, dans laquelle la maladie auto-immune ou allo-immune est une spondylarthrite ankylosante.

13. Composition pharmaceutique à utiliser selon la revendication 1, dans laquelle la maladie auto-immune ou allo-immune est une hépatite auto-immune.

14. Composition pharmaceutique à utiliser selon la revendication 1, dans laquelle la maladie auto-immune ou allo-immune est une réaction du greffon contre l'hôte ; dans laquelle la réaction du greffon contre l'hôte est une réaction aiguë du greffon contre l'hôte, ou une réaction chronique du greffon contre l'hôte.

15. Composition pharmaceutique à utiliser selon la revendication 1, dans laquelle la maladie auto-immune ou allo-immune est un rejet de greffe d'organe.

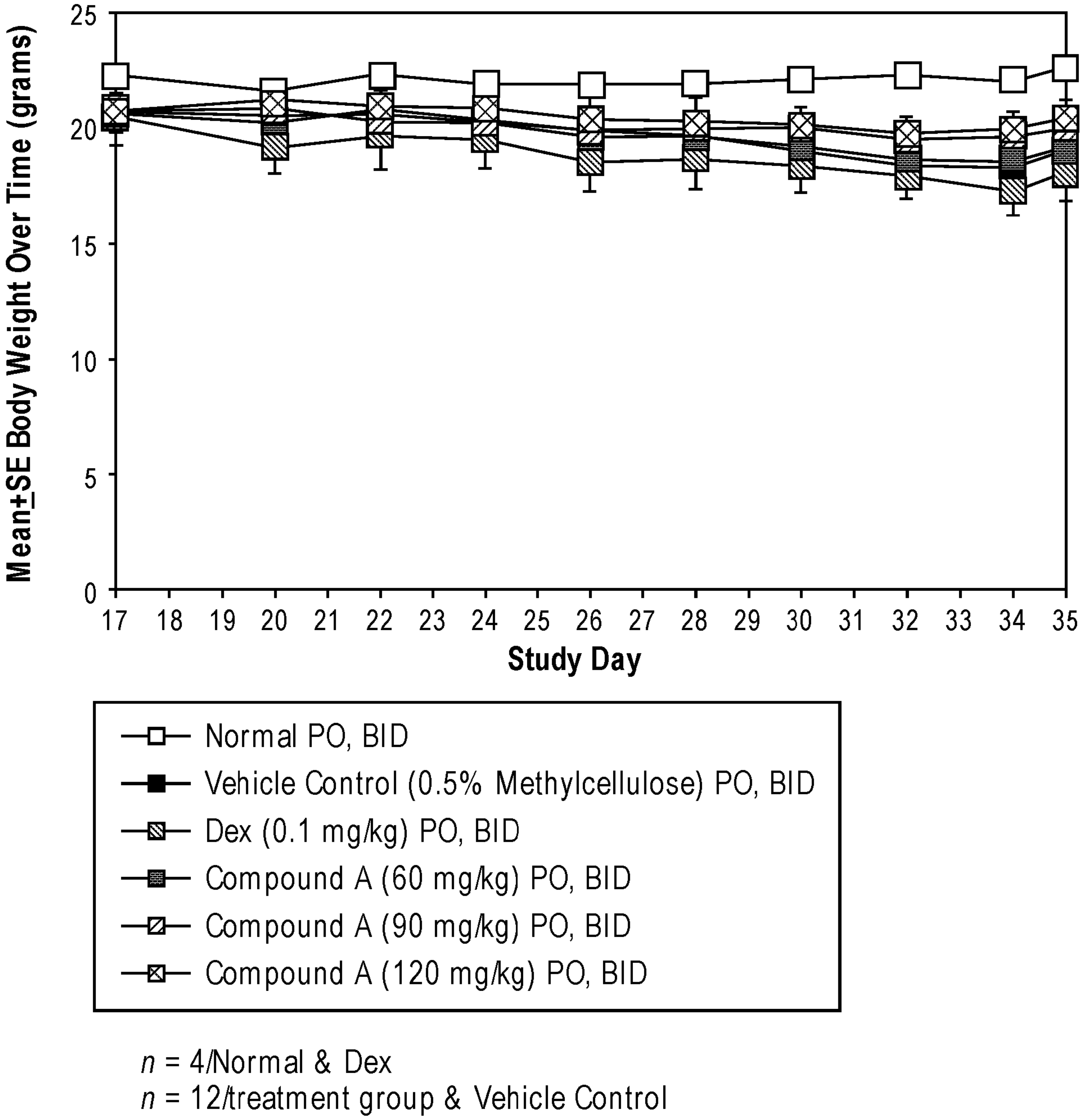
Mean±SE Body Weight Over Time (grams)
25
20
15
10
5
0
17 18 19 20 21 22 23 24 25 26 27 28 29 30 31 32 33 34 35
Study Day
Normal PO, BID
Vehicle Control (0.5% Methylcellulose) PO, BID
Dex (0.1 mg/kg) PO, BID
Compound A (60 mg/kg) PO, BID
Compound A (90 mg/kg) PO, BID
Compound A (120 mg/kg) PO, BID
n = 4/Normal & Dex
n = 12/treatment group & Vehicle Control

# FIG. 1

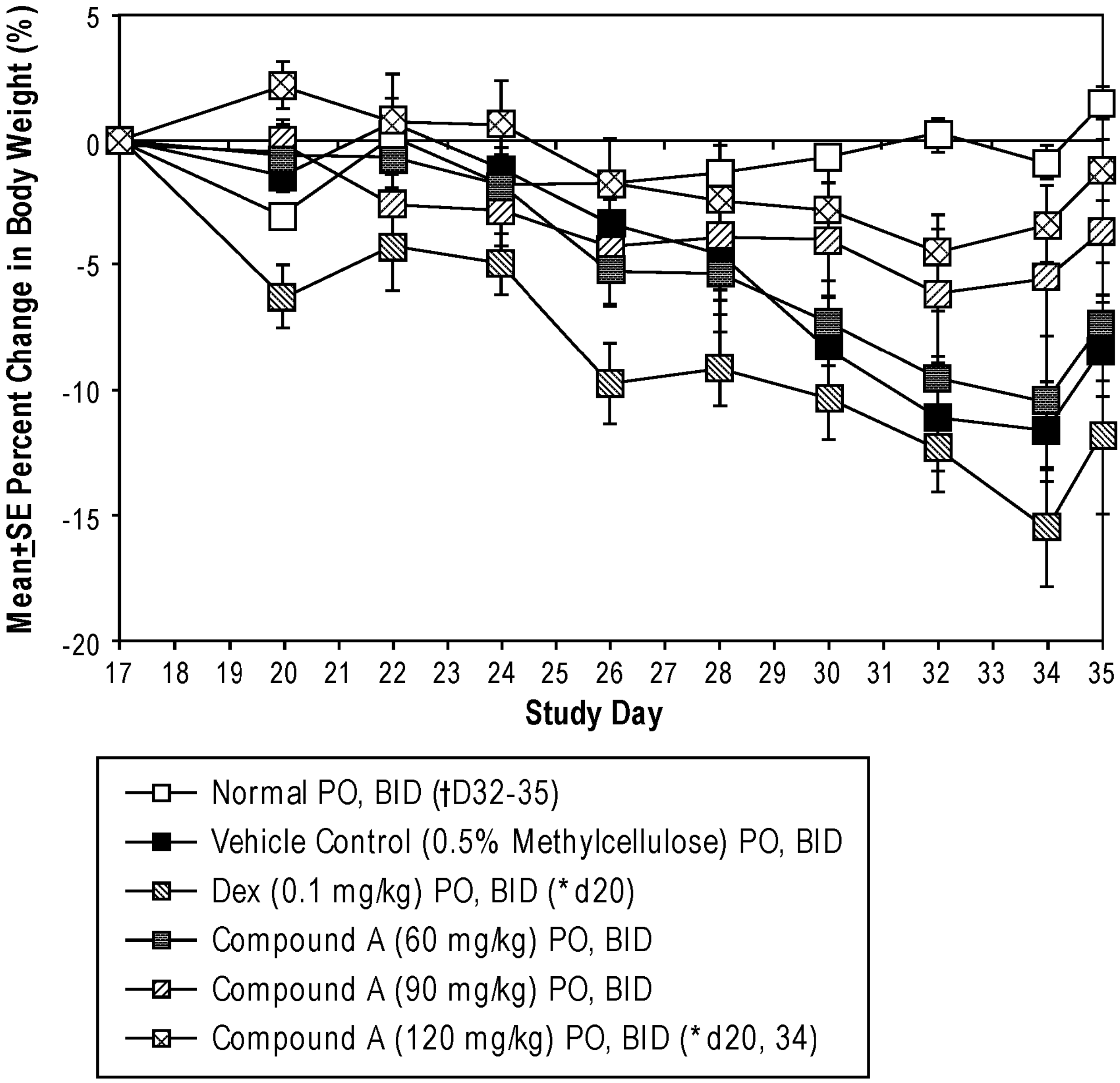


*n* = 4/Normal & Dex
*n* = 12/treatment group & Vehicle Control
**p* < 0.05 ANOVA vs. Vehicle Control
†*p* <0.05 *t*-test vs. Vehicle Control (comparison to Normal)

# FIG. 2

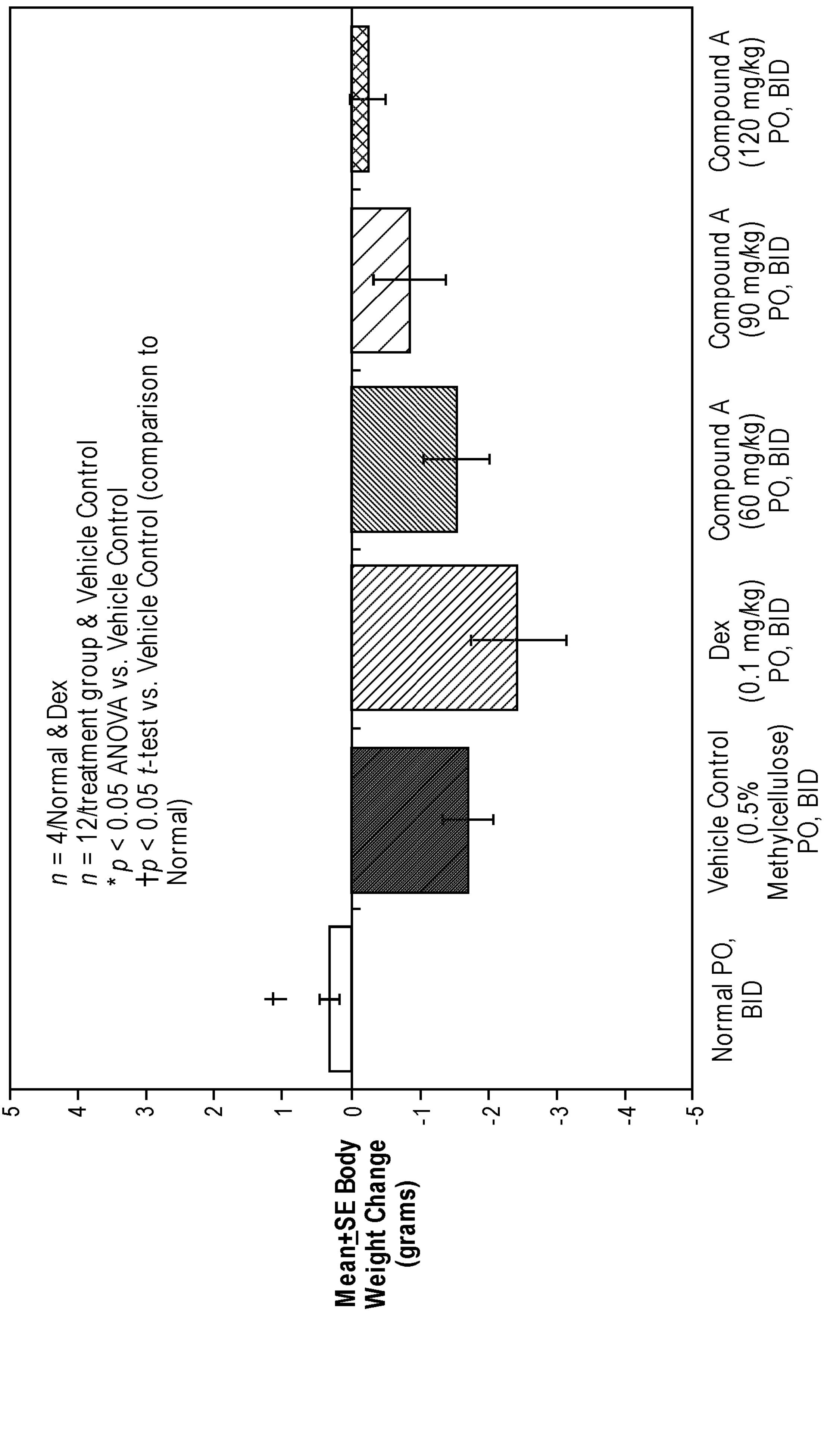
Mean±SE Body Weight Change (grams)
5
4
3
2
1
0
-1
-2
-3
-4
-5
n = 4/Normal & Dex
n = 12/treatment group & Vehicle Control
* p < 0.05 ANOVA vs. Vehicle Control
+p < 0.05 t-test vs. Vehicle Control (comparison to Normal)
+
Normal PO, BID
Vehicle Control (0.5% Methylcellulose) PO, BID
Dex (0.1 mg/kg) PO, BID
Compound A (60 mg/kg) PO, BID
Compound A (90 mg/kg) PO, BID
Compound A (120 mg/kg) PO, BID

FIG. 3

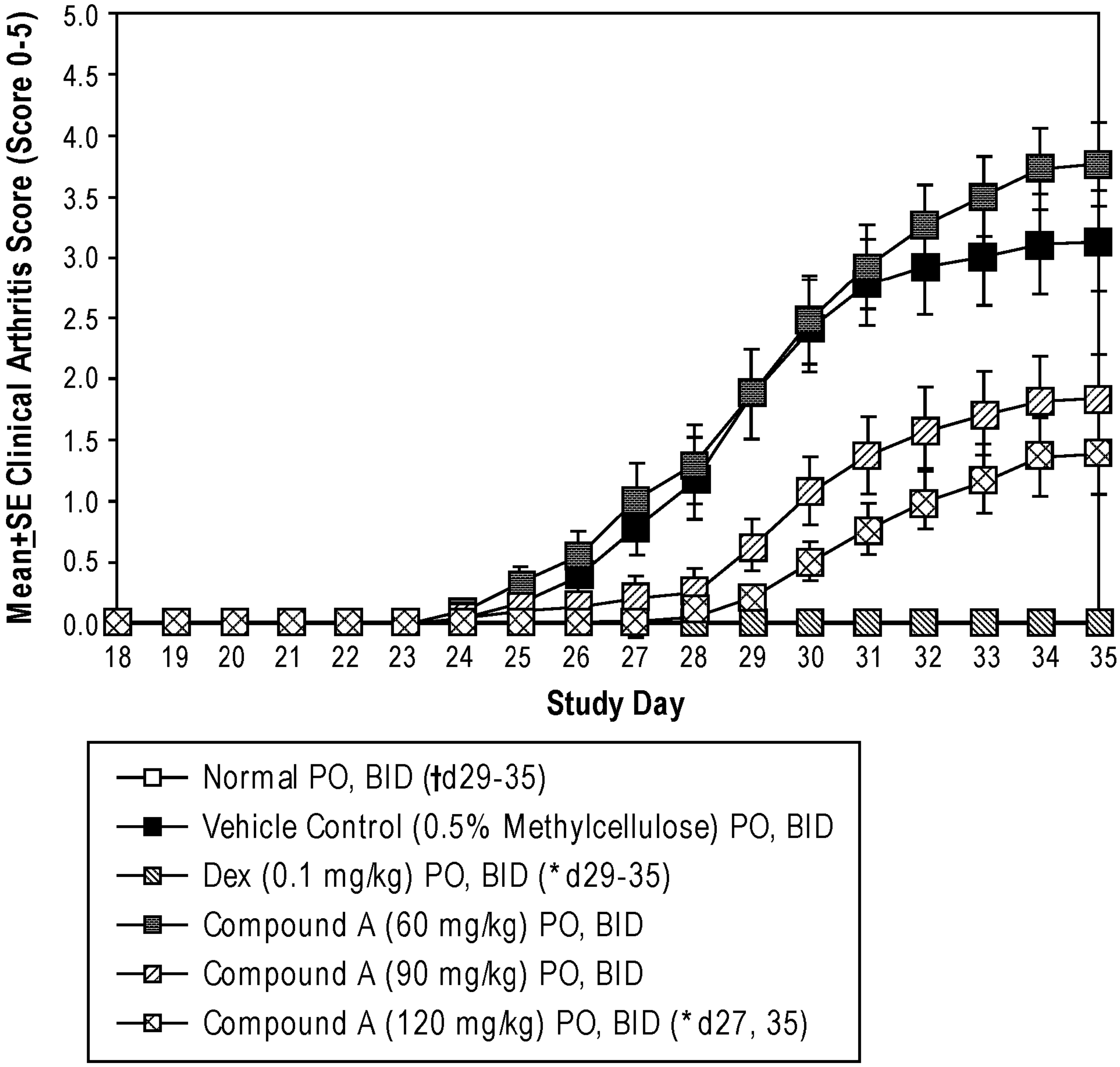


*n* = 4/Normal & Dex
*n* = 12/treatment group & Vehicle Control
**p* < 0.05 ANOVA vs. Vehicle Control
†*p* <0.05 *t*-test vs. Vehicle Control (comparison to Normal)

**FIG. 4**

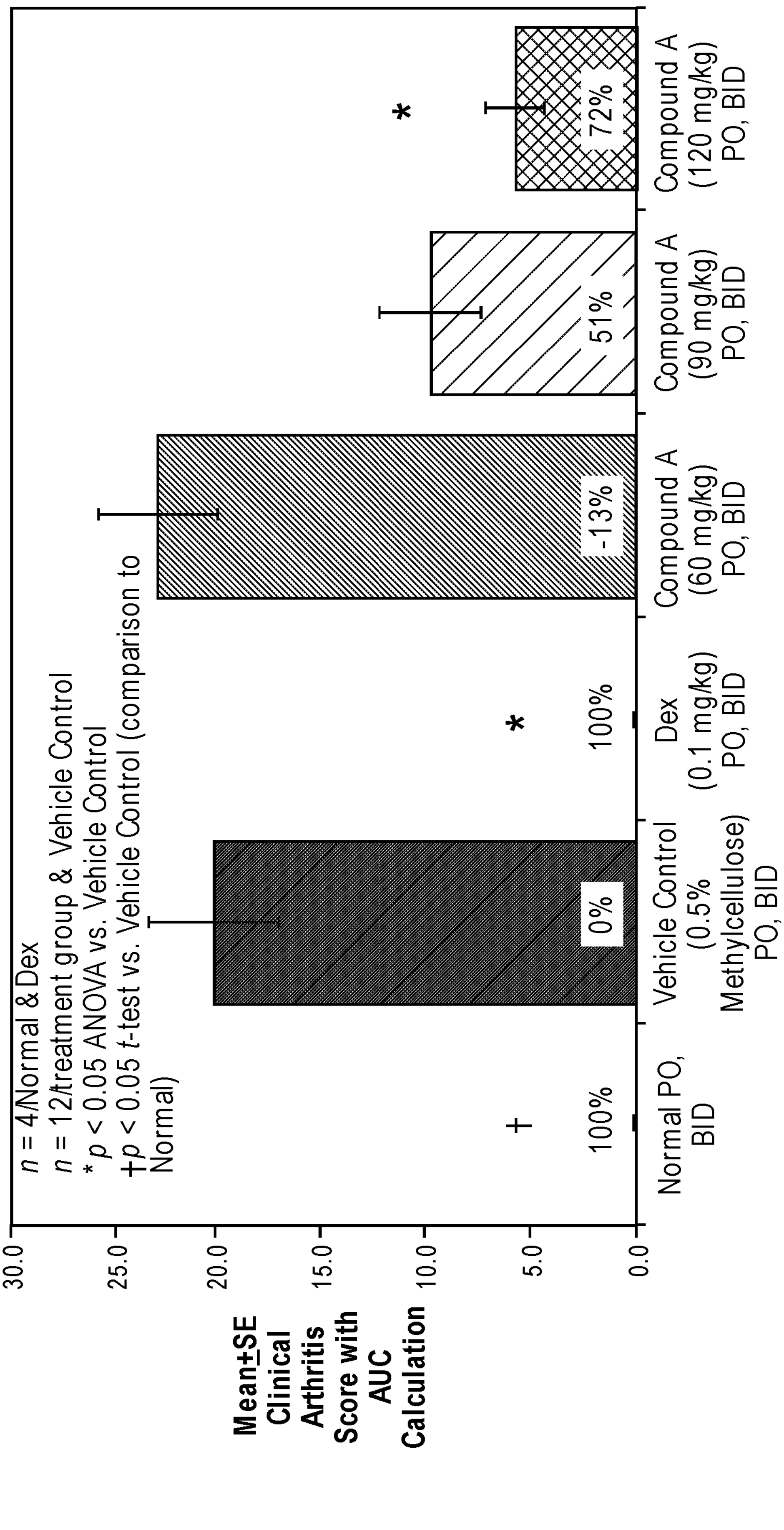

Mean±SE Clinical Arthritis Score with AUC Calculation
30.0
25.0
20.0
15.0
10.0
5.0
0.0
n = 4/Normal & Dex
n = 12/treatment group & Vehicle Control
* p < 0.05 ANOVA vs. Vehicle Control
+p < 0.05 t-test vs. Vehicle Control (comparison to Normal)
+
100%
0%
*
100%
-13%
51%
*
72%
Normal PO, BID
Vehicle Control (0.5% Methylcellulose) PO, BID
Dex (0.1 mg/kg) PO, BID
Compound A (60 mg/kg) PO, BID
Compound A (90 mg/kg) PO, BID
Compound A (120 mg/kg) PO, BID


FIG. 5

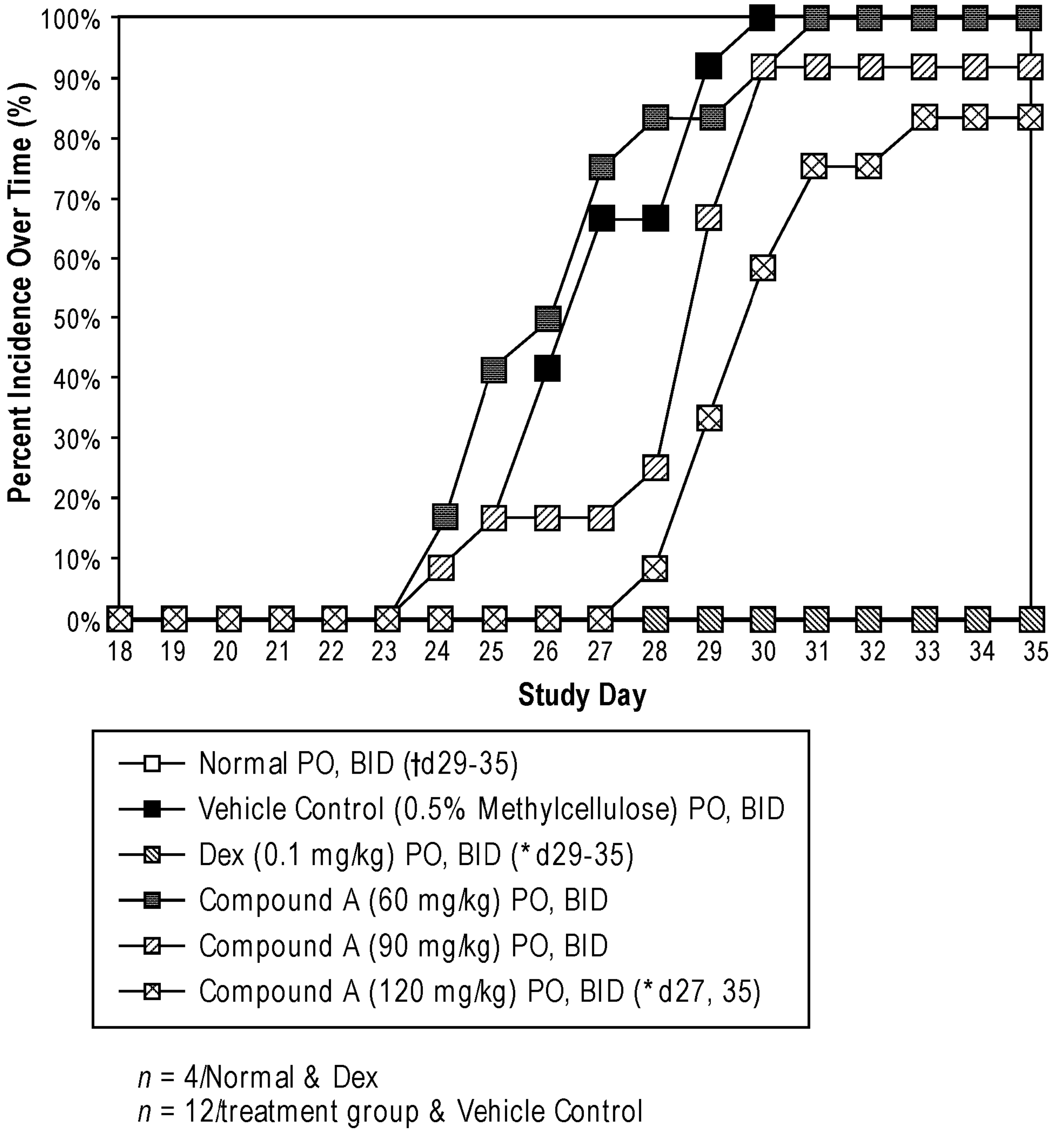
100%
90%
80%
70%
60%
50%
40%
30%
20%
10%
0%
Percent Incidence Over Time (%)
18 19 20 21 22 23 24 25 26 27 28 29 30 31 32 33 34 35
Study Day
Normal PO, BID (†d29-35)
Vehicle Control (0.5% Methylcellulose) PO, BID
Dex (0.1 mg/kg) PO, BID (* d29-35)
Compound A (60 mg/kg) PO, BID
Compound A (90 mg/kg) PO, BID
Compound A (120 mg/kg) PO, BID (* d27, 35)
n = 4/Normal & Dex
n = 12/treatment group & Vehicle Control

**FIG. 6**

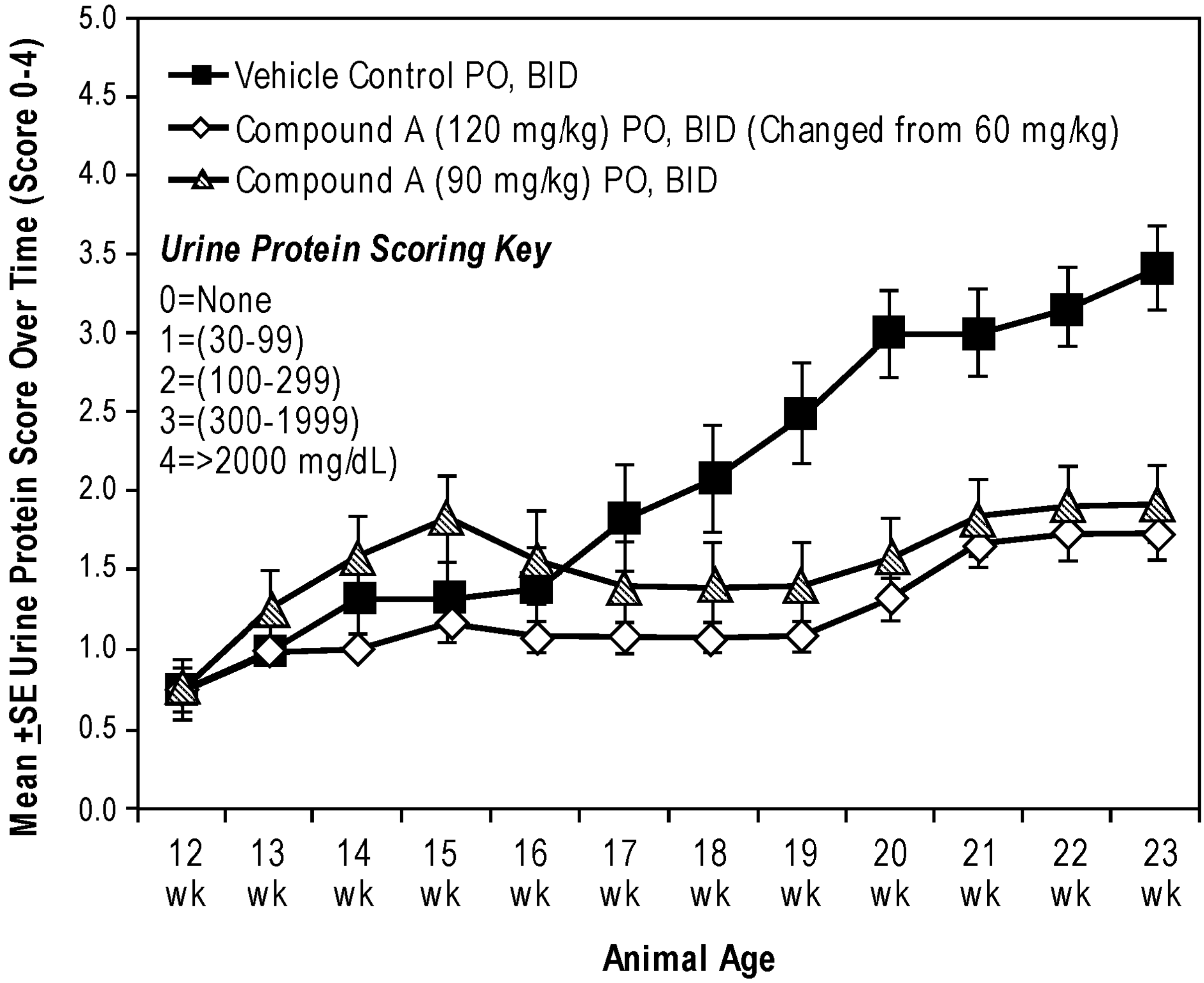
Vehicle Control PO, BID
Compound A (120 mg/kg) PO, BID (Changed from 60 mg/kg)
Compound A (90 mg/kg) PO, BID
Urine Protein Scoring Key
0=None
1=(30-99)
2=(100-299)
3=(300-1999)
4=>2000 mg/dL)
Mean ±SE Urine Protein Score Over Time (Score 0-4)
5.0
4.5
4.0
3.5
3.0
2.5
2.0
1.5
1.0
0.5
0.0
12 wk
13 wk
14 wk
15 wk
16 wk
17 wk
18 wk
19 wk
20 wk
21 wk
22 wk
23 wk
Animal Age

FIG. 7

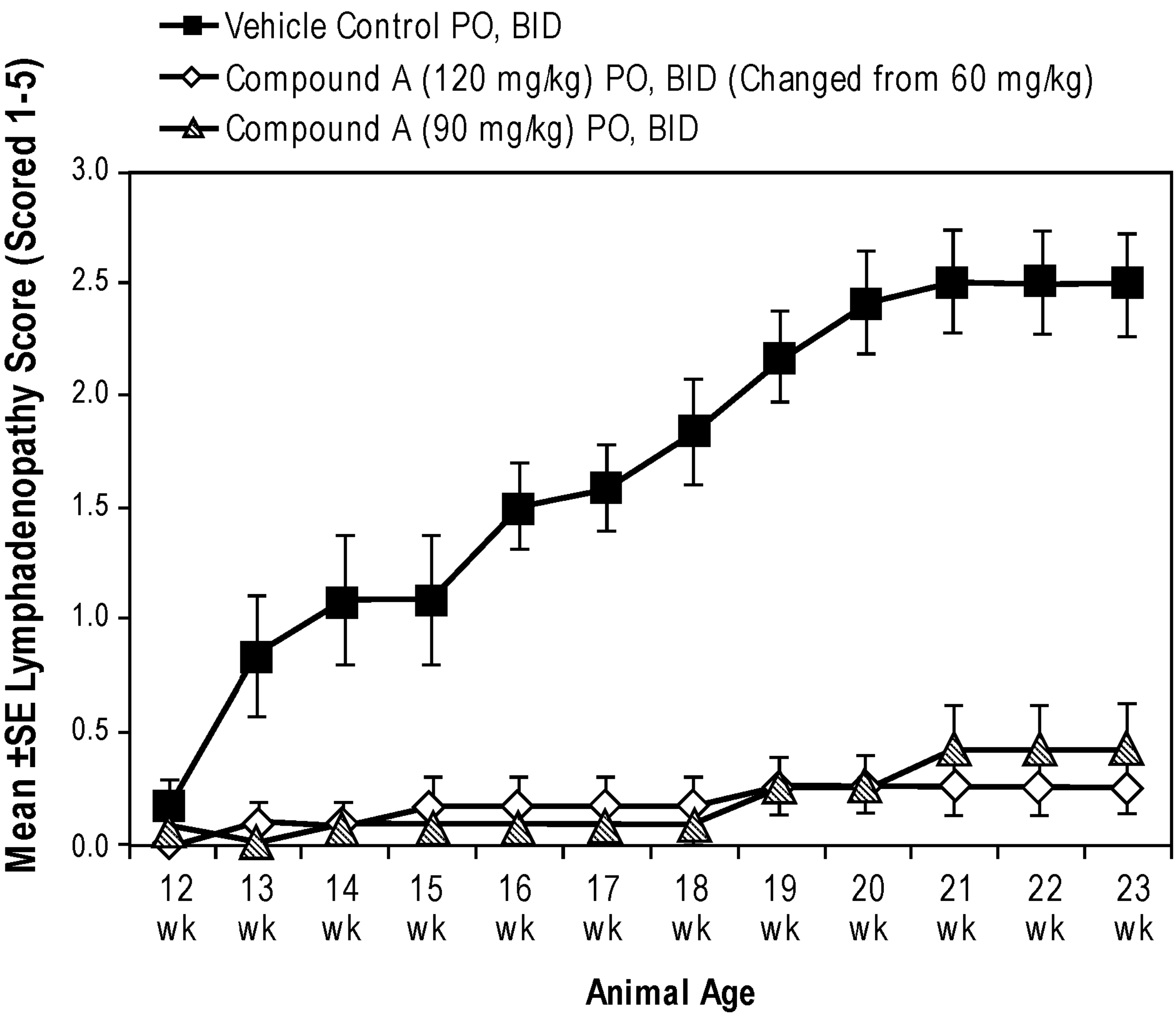
Vehicle Control PO, BID
Compound A (120 mg/kg) PO, BID (Changed from 60 mg/kg)
Compound A (90 mg/kg) PO, BID
Mean ±SE Lymphadenopathy Score (Scored 1-5)
3.0
2.5
2.0
1.5
1.0
0.5
0.0
12 wk
13 wk
14 wk
15 wk
16 wk
17 wk
18 wk
19 wk
20 wk
21 wk
22 wk
23 wk
Animal Age

FIG. 8

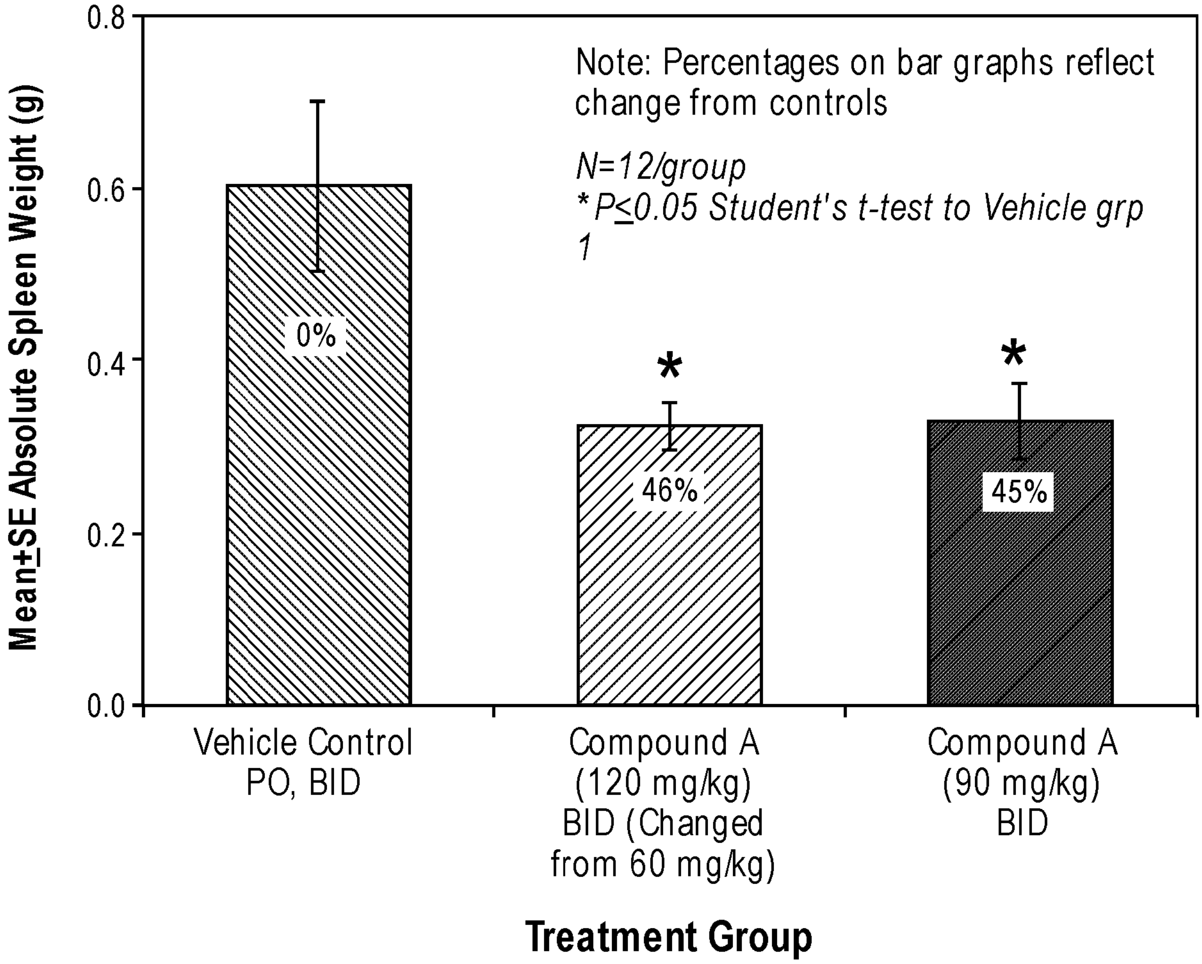
Mean±SE Absolute Spleen Weight (g)
0.8
0.6
0.4
0.2
0.0
Note: Percentages on bar graphs reflect change from controls
N=12/group
*P≤0.05 Student's t-test to Vehicle grp 1
0%
46%
45%
*
*
Vehicle Control PO, BID
Compound A (120 mg/kg) BID (Changed from 60 mg/kg)
Compound A (90 mg/kg) BID
Treatment Group

FIG. 9

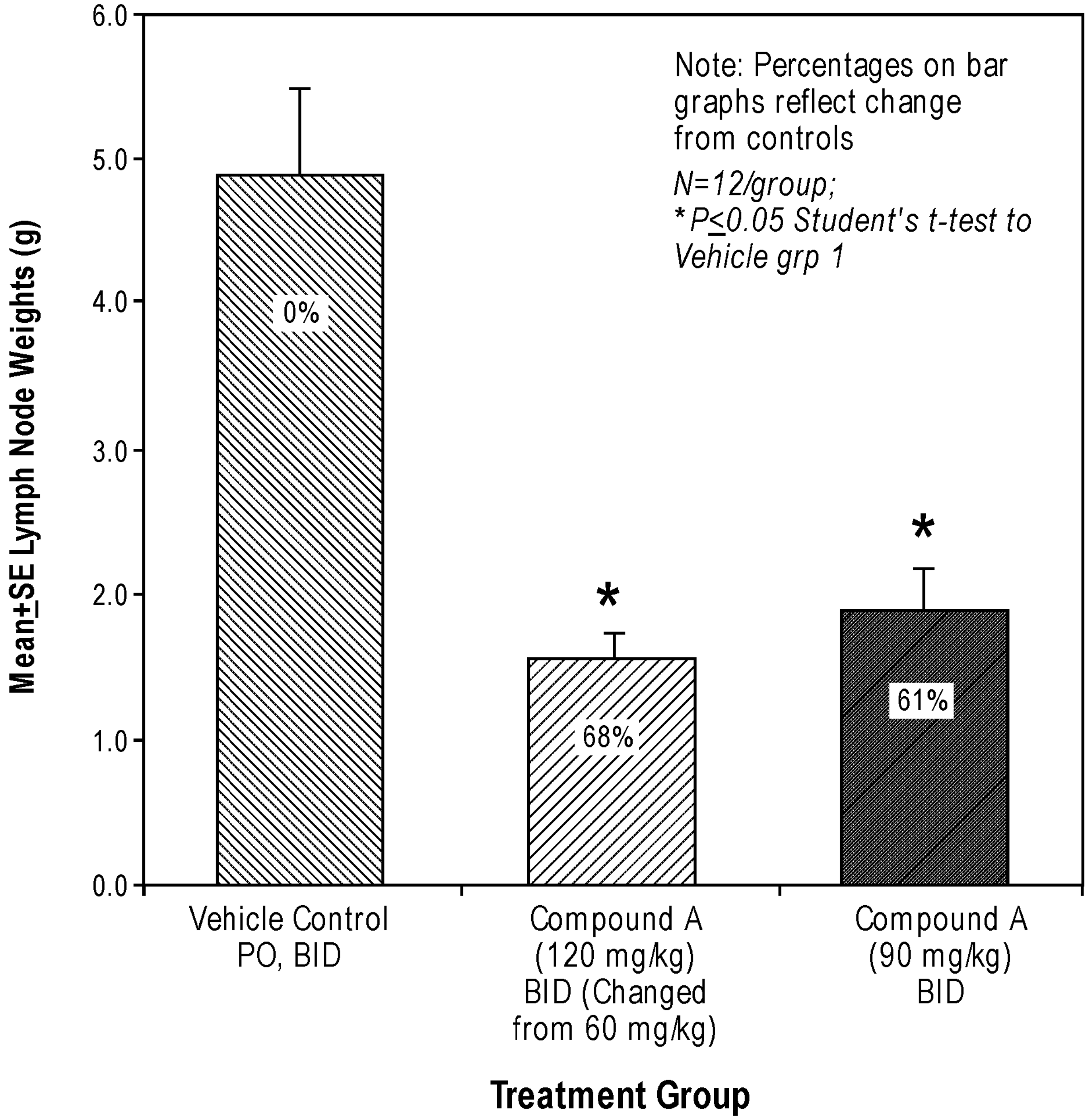
6.0
5.0
4.0
3.0
2.0
1.0
0.0
Mean±SE Lymph Node Weights (g)
Note: Percentages on bar graphs reflect change from controls
N=12/group;
*P≤0.05 Student's t-test to Vehicle grp 1
0%
68%
61%
*
*
Vehicle Control PO, BID
Compound A (120 mg/kg) BID (Changed from 60 mg/kg)
Compound A (90 mg/kg) BID
Treatment Group

FIG. 10

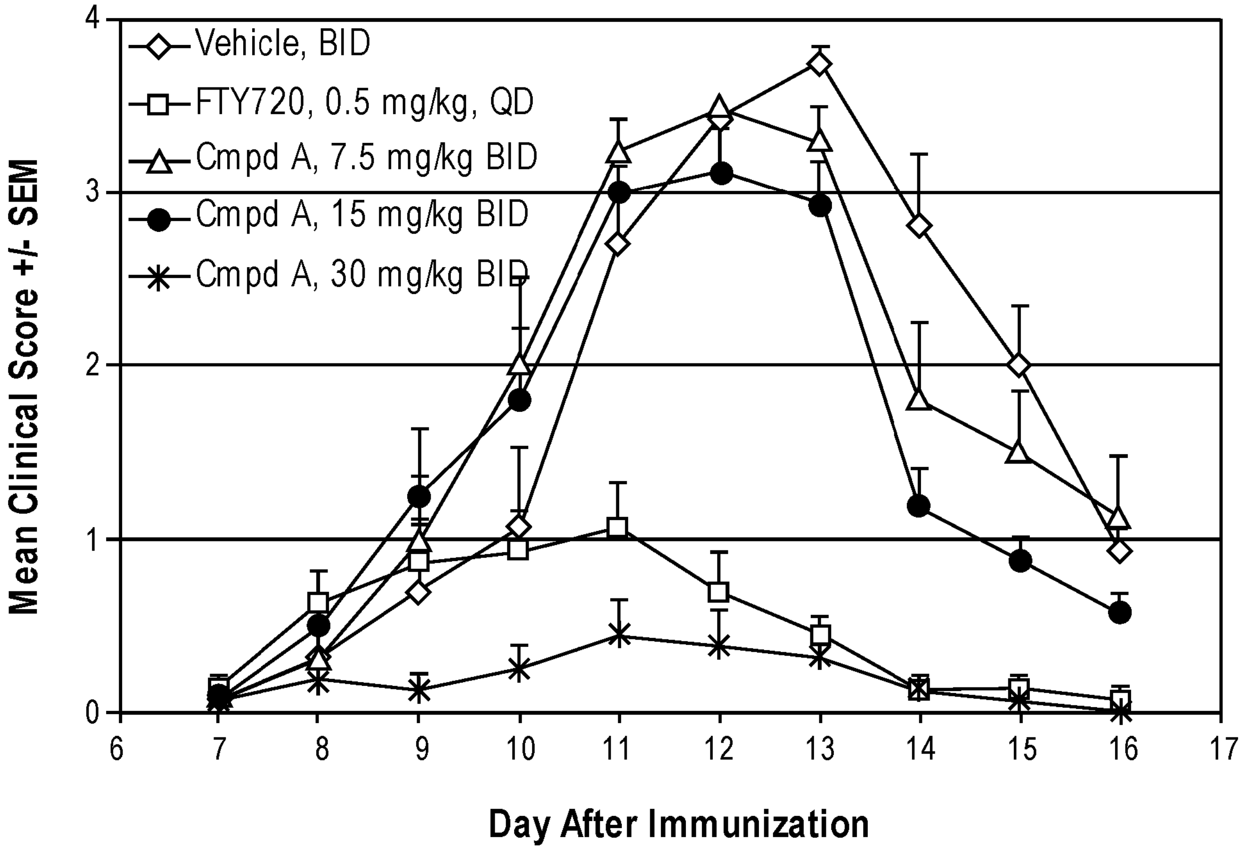
Mean Clinical Score +/- SEM
Day After Immunization
Vehicle, BID
FTY720, 0.5 mg/kg, QD
Cmpd A, 7.5 mg/kg BID
Cmpd A, 15 mg/kg BID
Cmpd A, 30 mg/kg BID
0
1
2
3
4
6
7
8
9
10
11
12
13
14
15
16
17

FIG. 11

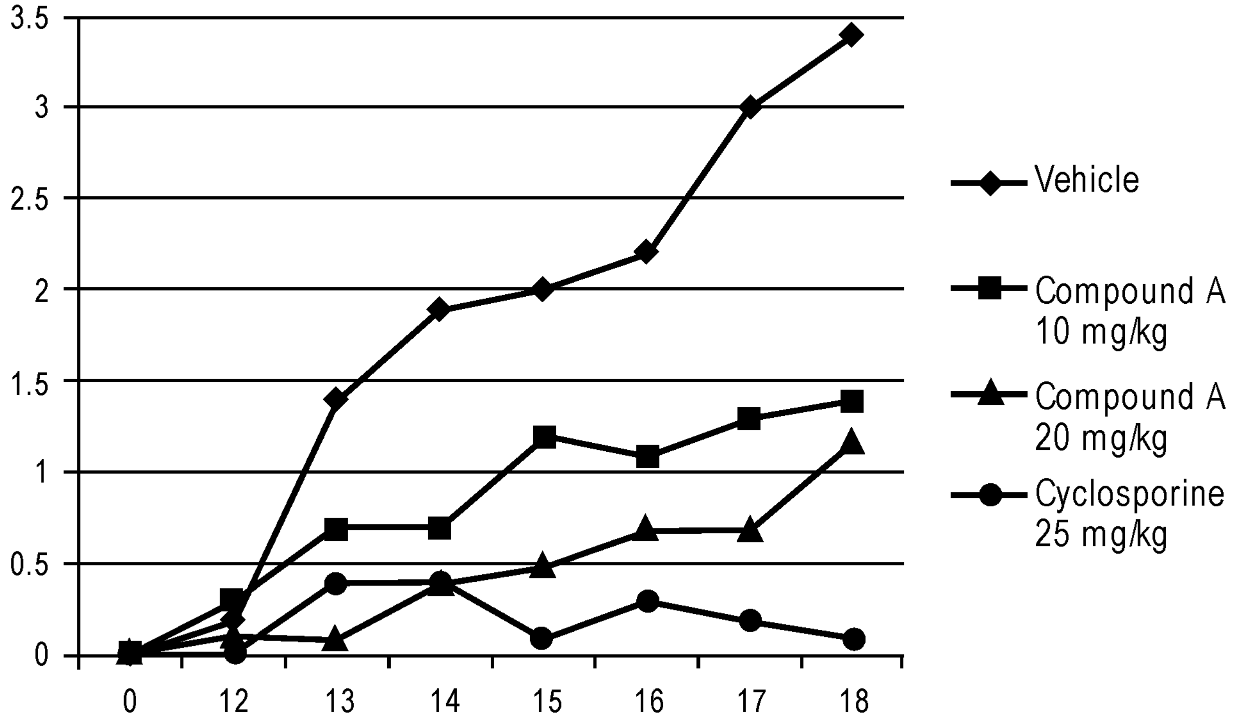
3.5
3
2.5
2
1.5
1
0.5
0
0
12
13
14
15
16
17
18
Vehicle
Compound A 10 mg/kg
Compound A 20 mg/kg
Cyclosporine 25 mg/kg

FIG. 12

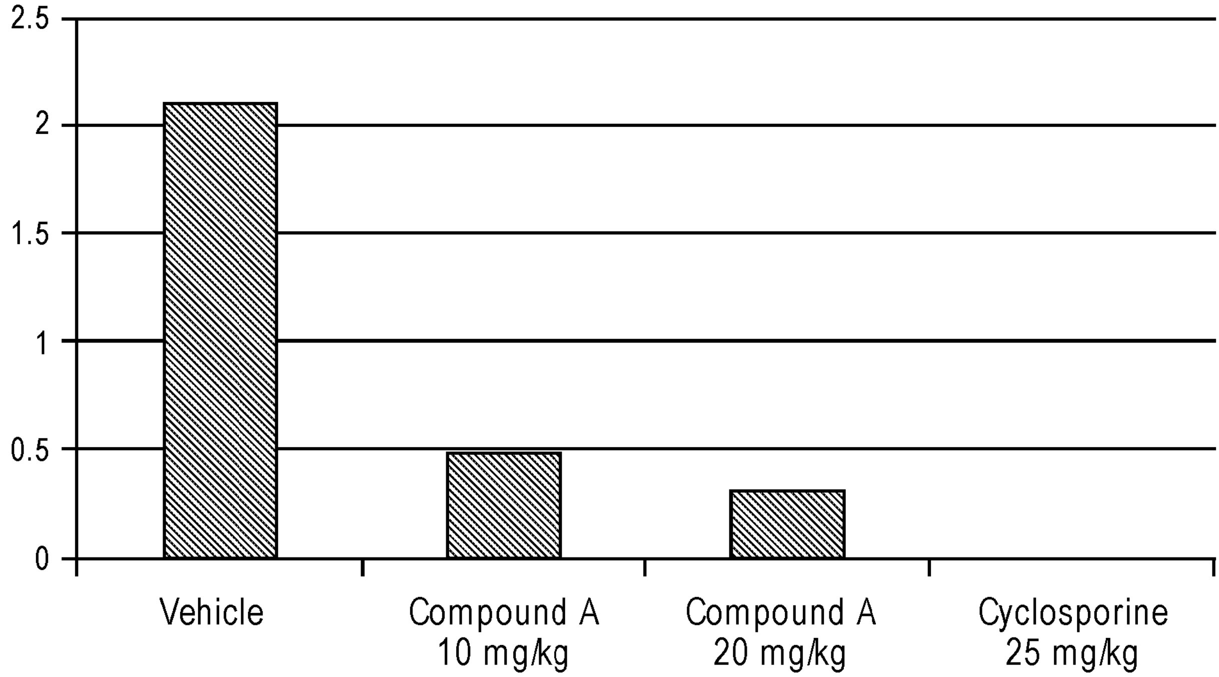
2.5
2
1.5
1
0.5
0
Vehicle
Compound A 10 mg/kg
Compound A 20 mg/kg
Cyclosporine 25 mg/kg

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20150099743 A1
- US 20070004705 A1
- WO 2008096260 A
- WO 2008125945 A
- US 8183255 B
- US 8877761 B
- US 506470
- US 8114871 B
- US 8999981 B


### Non-patent literature cited in the description


- **NEWTON.** *Chem. Rev.,* 2001, vol. 101, 2353-2364
- **EVCIMEN ; KING.** *Pharmacol Res,.,* 2007, vol. 55 (6), 498-510
- **SUN, Z.** *Front Immunol,* 2012, vol. 3, 225
- **LEITGES, M. et al.** *Science,* 1996, vol. 273, 788-791
- **GRUBER, T. et al.** *Mol Immunol,* 2009, vol. 46, 2071-2079
- **WAHREN-HERLENIUS ; DÖRNER T.** *Lancet,* 2013, vol. 382, 819-31
- **SUN.** *Front Immunol,* 2012, vol. 3, 225
- **MARSLAND, B.J. ; KOPF, M.** *Trends Immunol,* 2008, vol. 29 (4), 179-85
- **PFEIFHOFER, C. et al.** *J Immunol,* 2006, vol. 176, 6004-6011
- **ESSEN, M. et al.** *J Immunol,* 2006, vol. 176, 7502-75
- **SAIJO, K. et al.** *J Exp Med,* 2002, vol. 195, 1647-1652
- **SU, T.T. et al.** *Nat Immunol,* 2002, vol. 3, 780-786
- **MECKLENBRAUKER, I. et al.** *Nature,* 2002, vol. 416, 860-865
- **MIYAMOTO, A. et al.** *Nature,* 2002, vol. 416, 865-869
- **NISTALA et al.** Th17 plasticity in human autoimmune arthritis is driven by the inflammatory environment. *PNAS,* 2010, vol. 107 (33), 14751-14756
- **HEALY et al.** PKC-θ-deficient mice are protected from Th1-dependent antigen-induced arthritis. *J Immunol,* 2006, vol. 177, 1886-1893
- **COMPSTON, A ; COLES, A.** Multiple sclerosis. *Lancet,* 2008, vol. 372, 1502-1517
- **HOGLUND, R.A. ; MAGHAZACHI, A.A.** Multiple sclerosis and the role of immune cells. *World J. Exp Med.,* 2014, vol. 4 (3), 27-37
- **PRINEAS, J.W. ; GRAHAM, J.S.** Multiple sclerosis: capping of surface immunoglobulin G on macrophages engaged in myelin breakdown. *Ann Neurol,* 1981, vol. 10, 149-158
- **HESTVIK, A.L.** The double-edged sword of autoimmunity: lessons from multiple sclerosis. *Toxins,* 2010, vol. 2, 856-877
- **SALEK-ARDAKANI et al.** Protein kinase Cθ controls Th1 cells in experimental autoimmune encephalomyelitis. *J Immunol,* 2005, vol. 175, 7635-7641
- **ANDERSON et al.** Mice deficient in PKC theta demonstrate impaired in vivo T cell activation and protection from T cell-mediated inflammatory diseases. *Autoimmunity,* 2006, vol. 39 (6), 469-478
- **TAN et al.** Resistance to experimental autoimmune encephalomyelitis and impaired IL-17 production in protein kinase C {theta}-deficient mice. *J. Immunol.,* 2006, vol. 176, 2872-2879
- **SALEK-ARDAKANI et al.** Protein kinase C{theta} controls Th1 cells in experimental autoimmune encephalomyelitis. *J. Immunol.,* 2005, vol. 175, 7635-7641
- **FANG et al.** Ameliorated ConA-induced hepatitis in the absence of PKC-theta. *PLoS ONE,* 2012, vol. 7 (2), e31174
- **SUN, Z.** Intervention of PKC-θ as an immunosuppressive regimen. *Frontiers in Immunology,* 2012, vol. 3 (225), 1-9
- **ANDERSON et al.** Mice deficient in PKC theta demonstrate impaired in vivo T cell activation and protection from T cell-mediated inflammatory diseases. *Autoimmunity,* 2006, vol. 39, 469-478
- **MANICASSAMY et al.** Protein kinase C-{theta}-mediated signals Enhance CD4+ T cell survival by up-regulating Bcl-xL. *J. Immunol.,* 2006, vol. 176, 6709-6716
- **GRUBER et al.** PKCθ cooperates with PKCα in alloimmune responses of T cells in vivo. *Molecular Immunology,* 2009, vol. 46, 2071-2079

- **SUN, Z.** Intervention of PKC-θ as an immunosuppressive regiment. *Frontiers in Immunology,* 2012, vol. 3 (225), 1-9
- **VALENZUELA et al.** PKCθ is required for alloreactivity and GVHD but not for immune responses toward leukemia and infection in mice. *The Journal of Clinical Investigation,* 2009, vol. 119 (12), 3774-3786
- **GRANT et al.** *Eur J Pharmacol,* 2010, vol. 627, 16-25
- **BANGERT et al.** Immunopathologic features of allergic contact dermatitis in humans: participation of plasmacytoid dendritic cells in the pathogenesis of the disease?. *J. Invest. Dermatol.,* 2003, vol. 121, 1409-1418
- *Eye,* 2004, vol. 18, 1066-1074
- **SHLOMCHIK MJ et al.** *J Exp Med,* 1994, vol. 180, 1295-1306
- **COHEN PL ; EISENBERG R. A.** *Annu Rev Immunol,* 1991, vol. 9, 243-69
- **HONIGBERG, L.A. et al.** *Proc Natl Acad Sci U S A.,* 2010, vol. 107, 13075-80
- **HASHIM et al.** *J Neurosci Res.,* 1986, vol. 16 (3), 467-78
- **NUSSENBLATT RB et al.** *J Clin Invest.,* 1981, vol. 67 (4), 1228-1231
- **MOCHIZUKI M et al.** *Invest Ophthalmol Vis Sci.,* 1985, vol. 26 (2), 226-232